(19) **Europäisches Patentamt / European Patent Office / Office européen des brevets**

(11) **EP 1 702 982 B1**

(12) **EUROPEAN PATENT SPECIFICATION**

(45) Date of publication and mention
of the grant of the patent:
**05.05.2010 Bulletin 2010/18**

(21) Application number: **04819413.8**

(22) Date of filing: **25.11.2004**

(51) Int Cl.:
*C12N 15/11* (2006.01)   *C12Q 1/68* (2006.01)
*G01N 33/53* (2006.01)   *A61K 39/395* (2006.01)
*A61P 35/00* (2006.01)

(86) International application number:
**PCT/JP2004/017499**

(87) International publication number:
**WO 2005/052156 (09.06.2005 Gazette 2005/23)**

(54) **METHOD OF DETECTING LIVER CANCER, DIAGNOSTIC FOR LIVER CANCER AND REMEDY FOR CANCER**

VERFAHREN ZUM NACHWEIS VON LEBERKREBS, DIAGNOSTIKUM FÜR LEBERKREBS SOWIE HEILMITTEL GEGEN KREBS

METHODE DE DETECTION, DIAGNOSTIC ET REMEDE CONTRE LE CANCER DU FOIE

(84) Designated Contracting States:
**AT BE BG CH CY CZ DE DK EE ES FI FR GB GR HU IE IS IT LI LU MC NL PL PT RO SE SI SK TR**

(30) Priority: **28.11.2003 JP 2003399331**
**01.12.2003 JP 2003401585**
**19.12.2003 JP 2003423237**

(43) Date of publication of application:
**20.09.2006 Bulletin 2006/38**

(73) Proprietor: **KANAGAWA ACADEMY OF SCIENCE AND TECHNOLOGY**
**Kanagawa 213-0012 (JP)**

(72) Inventors:
• **NAKAMURA, Koji**
**Fuchu-shi, Tokyo 1830033 (JP)**
• **ANZAI, Hiroko**
**Kawasaki-shi, Kanagawa 2130027 (JP)**
• **YANAI, Hiroyuki**
**Kawasaki-shi, Kanagawa 2160002 (JP)**
• **MIYAJIMA, Atsushi**
**Nerima-ku, Tokyo 1770054 (JP)**

(74) Representative: **Cripps, Joanna Elizabeth et al**
**Mewburn Ellis LLP**
**33 Gutter Lane**
**London**
**EC2V 8AS (GB)**

(56) References cited:
**EP-A2- 1 013 762     WO-A1-02/103033**
WO-A2-02/081625     WO-A2-2004/030615
WO-A2-2004/048938     JP-A- 8 509 358

• **LABORDA J ET AL: "dlk, a putative mammalian homeotic gene differentially expressed in small cell lung carcinoma and neuroendocrine tumor cell line" JOURNAL OF BIOLOGICAL CHEMISTRY, AMERICAN SOCIETY OF BIOLOCHEMICAL BIOLOGISTS, BIRMINGHAM,, US, vol. 268, no. 6, 25 February 1993 (1993-02-25), pages 3817-3829, XP002144816 ISSN: 0021-9258**

• **FLORIDON C ET AL: "Does fetal antigen 1 (FA1) identify cells with regenerative, endocrine and neuroendocrine potentials? A study of FA1 in embryonic, fetal, and placental tissue and in maternal circulation" DIFFERENTIATION, SPRINGER VERLAG, DE, vol. 66, no. 1, August 2000 (2000-08), pages 49-59, XP002223507 ISSN: 0301-4681**

• **JENSEN C.H. ET AL: 'Protein structure of fetal antigen 1 (FA1). A novel circulating human epidermal-growth-factzor-like protein expressed in neuroendocrine tumors and its relation to the gene products of dlk and pG2' EUR.J.BIOCHEM. vol. 225, no. 1, 1994, pages 83 - 92, XP002986911**

• **BALADRON V. ET AL: 'The EGF-like Homeotic Protein in dlk Affects Cell Growth and Interacts with Growth-Modulating Molecules in the Yeast Two-Hybrid System' BIOCHEM.BIOPHYS.RES.COMMUN. vol. 291, no. 2, 2002, pages 193 - 204, XP002986912**

EP 1 702 982 B1

**(Cont. next page)**

- LEE Y.L. ET AL: 'dlk, pG2 and Pref-1 mRNAs encode similar proteins belonging to the EGF-like superfamily. Identification of polymorphic variants of this RNA' BIOCHIM.BIOPHYS.ACTA. vol. 1261, no. 2, 1995, pages 223 - 232, XP009008393
- JENSEN C.H. ET AL: 'Fetal antigen 1, a member of the epidermal growth factor superfamily, in neurofibromas and serum from patiens with neurofibromatosis type 1' BR.J.DERMATOL. vol. 140, no. 6, 1999, pages 1054 - 1059, XP002986913
- TANIMIZU N. ET AL: 'Isolation of hepatoblasts based on the expression of Dlk/Pref-1' J.CELL SCI. vol. 116, no. 9, 2003, pages 1775 - 1786, XP002986914

SEQUENCE LISTING

<110> KANAGAWA ACADEMY OF SCIENCE AND TECHNOLOGY

<120> Method for detecting hepatic cancer, diagnostic drug for hepatic cancer and therapeutic agent for cancers

<130> JEC/FP6390371

<140> EP04819413.8<141> 2004-11-25

<150> PCT/JP2004/017499<151> 2004-11-25

<150> JP2003-399331<151> 2003-11-28

<150> JP2003-401585<151> 2003-12-01

<150> JP2003-423237<151> 2003-12-19

<160> 6

<170> PatentIn version 3.1

<210> 1
<211> 1553
<212> DNA
<213> Homo sapiens

<220>
<221> CDS
<222> (174)..(1322)
<223>

<400> 1

```
tctaaaggag gtggagagcg caccgcagcc cggtgcagcc cggtgcagcc ctggctttcc        60

cctcgctgcg gcccgtgccc cctttcgcgt ccgcaaccag aagcccagtg cggcgccagg       120

agccggaccc gcgcccgcac cgctcccggg accgcgaccc cggccgccca gag atg          176
                                                            Met
                                                            1

acc gcg acc gaa gcc ctc ctg cgc gtc ctc ttg ctc ctg ctg gct ttc        224
Thr Ala Thr Glu Ala Leu Leu Arg Val Leu Leu Leu Leu Leu Ala Phe
          5               10              15

ggc cac agc acc tat ggg gct gaa tgc ttc ccg gcc tgc aac ccc caa        272
Gly His Ser Thr Tyr Gly Ala Glu Cys Phe Pro Ala Cys Asn Pro Gln
         20              25              30

aat gga ttc tgc gag gat gac aat gtt tgc agg tgc cag cct ggc tgg        320
Asn Gly Phe Cys Glu Asp Asp Asn Val Cys Arg Cys Gln Pro Gly Trp
```

|  | 35 | | | | | 40 | | | | | 45 | | | |  |
|---|---|---|---|---|---|---|---|---|---|---|---|---|---|---|---|

```
cag ggt ccc ctt tgt gac cag tgc gtg acc tct ccc ggc tgc ctt cac          368
Gln Gly Pro Leu Cys Asp Gln Cys Val Thr Ser Pro Gly Cys Leu His
50              55              60              65

gga ctc tgt gga gaa ccc ggg cag tgc att tgc acc gac ggc tgg gac          416
Gly Leu Cys Gly Glu Pro Gly Gln Cys Ile Cys Thr Asp Gly Trp Asp
            70              75              80

ggg gag ctc tgt gat aga gat gtt cgg gcc tgc tcc tcg gcc ccc tgt          464
Gly Glu Leu Cys Asp Arg Asp Val Arg Ala Cys Ser Ser Ala Pro Cys
        85              90              95

gcc aac aac ggg acc tgc gtg agc ctg gac ggt ggc ctc tat gaa tgc          512
Ala Asn Asn Gly Thr Cys Val Ser Leu Asp Gly Gly Leu Tyr Glu Cys
        100             105             110

tcc tgt gcc ccc ggg tac tcg gga aag gac tgc cag aaa aag gac ggg          560
Ser Cys Ala Pro Gly Tyr Ser Gly Lys Asp Cys Gln Lys Lys Asp Gly
    115             120             125

ccc tgt gtg atc aac ggc tcc ccc tgc cag cac gga ggc acc tgc gtg          608
Pro Cys Val Ile Asn Gly Ser Pro Cys Gln His Gly Gly Thr Cys Val
130             135             140             145

gat gat gag ggc cgg gcc tcc cat gcc tcc tgc ctg tgc ccc cct ggc          656
Asp Asp Glu Gly Arg Ala Ser His Ala Ser Cys Leu Cys Pro Pro Gly
            150             155             160

ttc tca ggc aat ttc tgc gag atc gtg gcc aac agc tgc acc ccc aac          704
Phe Ser Gly Asn Phe Cys Glu Ile Val Ala Asn Ser Cys Thr Pro Asn
            165             170             175

cca tgc gag aac gac ggc gtc tgc act gac att ggg ggc gac ttc cgc          752
Pro Cys Glu Asn Asp Gly Val Cys Thr Asp Ile Gly Gly Asp Phe Arg
        180             185             190

tgc cgg tgc cca gcc ggc ttc atc gac aag acc tgc agc cgc ccg gtg          800
Cys Arg Cys Pro Ala Gly Phe Ile Asp Lys Thr Cys Ser Arg Pro Val
    195             200             205

acc aac tgc gcc agc agc ccg tgc cag aac ggg ggc acc tgc ctg cag          848
Thr Asn Cys Ala Ser Ser Pro Cys Gln Asn Gly Gly Thr Cys Leu Gln
210             215             220             225

cac acc cag gtg agc tac gag tgt ctg tgc aag ccc gag ttc aca ggt          896
His Thr Gln Val Ser Tyr Glu Cys Leu Cys Lys Pro Glu Phe Thr Gly
            230             235             240

ctc acc tgt gtc aag aag cgc gcg ctg agc ccc cag cag gtc acc cgt          944
Leu Thr Cys Val Lys Lys Arg Ala Leu Ser Pro Gln Gln Val Thr Arg
            245             250             255

ctg ccc agc ggc tat ggg ctg gcc tac cgc ctg acc cct ggg gtg cac          992
Leu Pro Ser Gly Tyr Gly Leu Ala Tyr Arg Leu Thr Pro Gly Val His
        260             265             270

gag ctg ccg gtg cag cag ccg gag cac cgc atc ctg aag gtg tcc atg         1040
```

EP 1 702 982 B1

```
Glu Leu Pro Val Gln Gln Pro Glu His Arg Ile Leu Lys Val Ser Met
    275                 280             285

aaa gag ctc aac aag aaa acc cct ctc ctc acc gag ggc cag gcc atc    1088
Lys Glu Leu Asn Lys Lys Thr Pro Leu Leu Thr Glu Gly Gln Ala Ile
290             295                 300                 305

tgc ttc acc atc ctg ggc gtg ctc acc agc ctg gtg gtg ctg ggc act    1136
Cys Phe Thr Ile Leu Gly Val Leu Thr Ser Leu Val Val Leu Gly Thr
            310                 315                 320

gtg ggt atc gtc ttc ctc aac aag tgc gag acc tgg gtg tcc aac ctg    1184
Val Gly Ile Val Phe Leu Asn Lys Cys Glu Thr Trp Val Ser Asn Leu
            325                 330                 335

cgc tac aac cac atg ctg cgg aag aag aac ctg ctg ctt cag tac aac    1232
Arg Tyr Asn His Met Leu Arg Lys Lys Asn Leu Leu Leu Gln Tyr Asn
            340                 345                 350

agc ggg gag gac ctg gcc gtc aac atc atc ttc ccc gag aag atc gac    1280
Ser Gly Glu Asp Leu Ala Val Asn Ile Ile Phe Pro Glu Lys Ile Asp
            355                 360                 365

atg acc acc ttc agc aag gag gcc ggc gac gag gag atc taa           1322
Met Thr Thr Phe Ser Lys Glu Ala Gly Asp Glu Glu Ile
370             375                 380

gcagcgttcc cacagccccc tctagattct tggagttccg cagagcttac tatacgcggt    1382

ctgtcctaat ctttgtggtg ttcgctatct cttgtgtcaa atctggtgaa cgctacgctt    1442

acatatattg tctttgtgct gctgtgtgac aaacgcaatg caaaaacaat cctctttctc    1502

tctcttaatg catgatacag aataataata agaatttcat ctttaaatga g           1553
```

<210> 2
<211> 382
<212> PRT
<213> Homo sapiens

<400> 2

```
Met Thr Ala Thr Glu Ala Leu Leu Arg Val Leu Leu Leu Leu Leu Ala
1               5                   10                  15

Phe Gly His Ser Thr Tyr Gly Ala Glu Cys Phe Pro Ala Cys Asn Pro
            20                  25                  30

Gln Asn Gly Phe Cys Glu Asp Asp Asn Val Cys Arg Cys Gln Pro Gly
            35                  40                  45

Trp Gln Gly Pro Leu Cys Asp Gln Cys Val Thr Ser Pro Gly Cys Leu
        50                  55                  60
```

21

His Gly Leu Cys Gly Glu Pro Gly Gln Cys Ile Cys Thr Asp Gly Trp
65              70              75              80

Asp Gly Glu Leu Cys Asp Arg Asp Val Arg Ala Cys Ser Ser Ala Pro
            85              90              95

Cys Ala Asn Asn Gly Thr Cys Val Ser Leu Asp Gly Gly Leu Tyr Glu
        100             105             110

Cys Ser Cys Ala Pro Gly Tyr Ser Gly Lys Asp Cys Gln Lys Lys Asp
        115             120             125

Gly Pro Cys Val Ile Asn Gly Ser Pro Cys Gln His Gly Gly Thr Cys
    130             135             140

Val Asp Asp Glu Gly Arg Ala Ser His Ala Ser Cys Leu Cys Pro Pro
145             150             155             160

Gly Phe Ser Gly Asn Phe Cys Glu Ile Val Ala Asn Ser Cys Thr Pro
            165             170             175

Asn Pro Cys Glu Asn Asp Gly Val Cys Thr Asp Ile Gly Gly Asp Phe
        180             185             190

Arg Cys Arg Cys Pro Ala Gly Phe Ile Asp Lys Thr Cys Ser Arg Pro
    195             200             205

Val Thr Asn Cys Ala Ser Ser Pro Cys Gln Asn Gly Gly Thr Cys Leu
    210             215             220

Gln His Thr Gln Val Ser Tyr Glu Cys Leu Cys Lys Pro Glu Phe Thr
225             230             235             240

Gly Leu Thr Cys Val Lys Lys Arg Ala Leu Ser Pro Gln Gln Val Thr
            245             250             255

Arg Leu Pro Ser Gly Tyr Gly Leu Ala Tyr Arg Leu Thr Pro Gly Val
        260             265             270

His Glu Leu Pro Val Gln Gln Pro Glu His Arg Ile Leu Lys Val Ser
        275             280             285

Met Lys Glu Leu Asn Lys Lys Thr Pro Leu Leu Thr Glu Gly Gln Ala
    290             295             300

```
        Ile Cys Phe Thr Ile Leu Gly Val Leu Thr Ser Leu Val Val Leu Gly
        305             310             315                 320


        Thr Val Gly Ile Val Phe Leu Asn Lys Cys Glu Thr Trp Val Ser Asn
                        325             330                 335


        Leu Arg Tyr Asn His Met Leu Arg Lys Lys Asn Leu Leu Leu Gln Tyr
                    340             345             350


        Asn Ser Gly Glu Asp Leu Ala Val Asn Ile Ile Phe Pro Glu Lys Ile
                355             360             365


        Asp Met Thr Thr Phe Ser Lys Glu Ala Gly Asp Glu Glu Ile
            370             375             380
```

<210> 3
<211> 21
<212> DNA
<213> Homo sapiens

<220>
<221> misc_feature
<223> synthetic oligoDNA primer used for amplification of human dlkcDN A

<400> 3
cgcgtccgca accagaagcc c          21

<210> 4
<211> 25
<212> DNA
<213> Homo sapiens

<220>
<221> misc_feature
<223> synthetic oligoDNA primer used for amplification of human dlkcDN A

<400> 4
aagcttgatc tcctcgtcgc cggcc          25

<210> 5
<211> 19
<212> DNA
<213> Homo sapiens

<220>
<221> misc feature
<223> synthetic oligoDNA primer used for amplification of human dlk cDN A

<400> 5
agagctcaac aagaaaacc          19

<210> 6
<211> 20
<212> DNA
<213> Homo sapiens

<220>
<221> misc feature
<223> synthetic oligoDNA primer used for amplification of human dlk cDN A

<400> 6
gcgtatagta agctctgagg          20

## Description

Technical Field

The present invention relates to a method for detecting liver cancer, diagnostic for liver cancer, and to a therapeutic drug for cancer.

Background Art

Hepatocellular carcinoma is one of the most popular carcinomas in the world, and onset thereof is especially frequent in South East Asia, China, and sub-Saharan Africa. Not less than 30,000 people die for liver cancer in Japan per year, and the number of deaths is still increasing. Most of the liver cancer is hepatocellular carcinoma caused by infection with hepatitis virus. However, the canceration mechanism from viral hepatitis to hepatocellular carcinoma through cirrhosis is still unclear. Therefore, presently used diagnostic methods (ultrasonography, diagnostic imaging by CT, hemodiagnosis using a tumor marker such as $\alpha$-fetoprotein) are those targeting already formed cancer tissues. Thus, although they can detect cancers which have progressed to some degree, they cannot detect cancer cells in a very early stage or precancerous cells. Although the hemodiagnosis using AFP as a tumor marker is simple, the specificity to liver cancer is not high, and it is known that AFP level is also high in cirrhosis and hepatitis.

The mortality rate of cancer in Japan increased from about 1980 and cancer is now the leading cause of death. Among the cancers, the number of death from liver cancer is 35,000 per year, which is the third position among the total death by cancers. It is thought that the number of patients of liver cancer will further increase unless an epoch-making diagnostic and therapeutic drug are developed. Current therapies of liver cancer include local treatments such as surgical hepatectomy, percutaneous ethanol-infusion therapy and hepatic arterial embolization, and systemic treatments such as systemic administration of anticancer agents and immunotherapies. The major therapies are local treatments, and hepatectomy is better than percutaneous ethanol-infusion therapy and hepatic arterial embolization in view of cure rate. However, depending on the degree of dysfunction of the liver and on the area occupied by the tumor, surgery often cannot be adopted. As for the systemic treatments, standard chemotherapy has not been established. Cisplatin is the only drug which exhibited a response rate of not less than 10% when administered alone, and polypharmacy has not been established (Non-patent Literature 1). As for immunotherapy, it has been reported that "Picibanil (OK-432)" (CHUGAI PHARMACEUTICAL) which is an immunostimulant is effective for liver cancer. Even if such a therapy is applied, complete cure of liver cancer is difficult because of its multicentric carcinogenesis and recurrent nature. It is thought that development of a molecularly targeted drug (therapeutic antibody) which specifically attacks liver cancer is important.

In recent several years, marketing and development of molecularly targeted drugs which specifically attack cancer cells are more and more active. Since these drugs target a target gene specifically expressed in a specific cancer, they have advantages that they are more effective than the conventional anticancer agents and they have less side effects. Therefore, it is thought that molecularly targeted drugs will become the mainstream of development of anticancer agents. Commercialized therapeutic antibodies for cancers include "Herceptin (anti-Her2 humanized monoclonal antibody preparation)" (CHUGAI PHARMACEUTICAL) which is a therapeutic drug for metastatic breast cancer in which excess expression of Her2 is confirmed, and "Rituxan (anti-CD20 chimeric monoclonal antibody preparation) (CHUGAI PHARMACEUTICAL and ZENYAKU KOGYO) which is a therapeutic drug for CD20-positive B-cell type non-Hodgkin lymphoma. These therapeutic antibodies kill cancer cells by immune mechanism such as antibody-dependent cell-mediated cytotoxicity, ADCC) or complement-dependent cytotoxicity, CDC). Although the number of commercialized cancer-specific molecularly targeted drugs is small, it is expected that the cure rate of cancers including liver cancer will be increased if drug products having a high specificity to a cancer are developed.

On the other hand, Dlk1/Pref-1 is a membrane protein whose extracellular domain has a homology with Notch/Delta/Serrate family. Dlk1/Pref-1 was cloned as a molecule expressing on a cell line derived from lung small cell carcinoma responsive to GRP (gastrin releasing peptide) (Non-patent Literature 1) or as a factor inhibiting differentiation of preadipocyte (Non-patent Literature 2). Its expression is observed in a plurality of tissues and organs during fetal period, but not observed in most of tissues after birth (Non-patent Literatures 2 and 3). Further, its expression is observed in some cancer tissues such as lung small cell carcinoma and type 1 neurofibromatosis (Non-patent Literatures 4 and 5). As for the function of Dlk1/Pref-1, in addition to the inhibition of differentiation of preadipocyte, participation in hematopoiesis was suggested recently (Non-patent Literature 6). However, based on the expression pattern and the like, the possibility of participating in maintaining undifferentiated state in undifferentiated cells has been suggested.

We previously identified dlk gene which was highly expressed in the liver of mouse at embryonic day 14.5, by the signal trap method that selectively isolates genes encoding molecules having a signal sequence, that is, those encoding cell surface antigens and secretory proteins. Expression of Dlk in the developmental process of mouse liver is observed before embryonic day 10, and it is strongly expressed until around embryonic day 16. However, the expression is dramatically decreased around the birth, and is not expressed in the mature liver (Non-patent Literatures 7 and 13).

Further, we discovered that hepatic stem cells may be purified to a high purity in one step from fetal liver using an anti-Dlk monoclonal antibody (Non-patent Literature 7, Patent Literature 1).

Non-patent Literature 1: Laborda, J., et al (1993) J. Biol .Chem. 268(6):3817-20
Non-patent Literature 2: Smas, C.M., et al (1993) Cell. 73(4):725-34
Non-patent Literature 3: Floridon, C., et al (2000) Differentiation 66(1):49-59
Non-patent Literature 4: Harken, J.C., et al (1999) Tumour Biol. 20(5):256-62
Non-patent Literature 5: Jensen, C.H., et al (1999) Br. J. Dermatol. 140(6): 1054-9
Non-patent Literature 6: Ohno, N., et al (2001) Stem Cells 19(1):71-9
Non-patent Literature 7: Tanimizu, N., et al (2003) J. Cell Sci. 116(Pt 9):1775-86
Non-patent Literature 8: Onishi, M., et al (1996) Exp. Hematol. 24;324-329
Non-patent Literature 9: Sell, S. (1993) Int. J. Dev. Biol. 37:189-201
Non-patent Literature 10: Jensen, C.H. et al (1994) Eur. J. Biochem. 225:83-92
Non-patent Literature 11: Kaneta, M. et al. (2000) J. Immunol. 164:256-264
Non-patent Literature 12: Okada, S., et al (1993) Oncology. 50 (1): 22-26.
Non-patent Literature 13: Kitajima, T., et al (1999) Nat. Biotechnol. 17 (5): 487-490.
Non-patent Literature 14: Jensen, C. H., et al (1999) Br. J. Dermatol. 140 (6): 1054-1059.
Non-patent Literature 15: Russell, W. C., et al (1977) J. Gen. Virol. 36: 59-72.
Non-patent Literature 16: Kipps, T. J., et al (1985) J. Exp. Med. 161: 1-17.
Patent Literature 1: International Patent Publication WO 02/103033

Disclosure of the Invention

Problems Which the Invention Tries to Solve

An object of the present invention is to provide a method for detecting liver cancer by which liver cancer may be detected with high specificity and to provide a diagnostic therefor. Another object of the present invention is to provide a novel therapeutic drug for cancer, which has an excellent anticancer effect.

Means for Solving the Problems

The present inventors intensively studied to discover that dlk is expressed on the surfaces of liver cancer cells of adults and experimentally confirmed that liver cancer cells may be detected using the dlk as a tumor marker. Further, the present inventors succeeded in preparing anti-human dlk monoclonal antibodies each of which undergoes antigen-antibody reaction with the extracellular domain of dlk expressing on cell surfaces, and confirmed that each of these anti-human dlk monoclonal antibodies also undergoes antigen-antibody reaction with FA1 which is the extracellular domain of dlk liberated into the blood.

The present inventors further inferred that there was a possibility that the anti-human dlk monoclonal antibody may be used as a therapeutic antibody targeting cancer cells expressing Dlk. Thus, the present inventors studied anti-tumor activities to specifically kill the cells of cancer cell lines expressing Dlk, of the prepared three anti-human Dlk monoclonal antibodies, using an *in vitro* experimental system, to confirm the anticancer activity of the anti-human Dlk monoclonal antibodies, thereby completing the present invention.

That is, the present invention provides use of an antibody which undergoes antigen-antibody reaction with Dlk expressing on surfaces of liver cancer cells and which exerts anticancer action against said liver cancer cells, for the production of a therapeutic drug for liver cancer.

Effects of the Invention

A method for detecting liver cancer, which utilizes a novel liver cancer marker is also described. Since dlk is not detected in organs other than placenta in adults and since dlk is also not detected in mouse acute liver injury models, liver cancer may be detected with high specificity n. Further, since dlk is expressed in the highly proliferative liver cells during fetal period and in oval cells emerging in regeneration of the liver in adults, it is thought that dlk is expressed in the growing liver cancer cells, so that it is thought that liver cancer at an early stage may be detected. Further, since FA1 which is the extracellular domain of dlk liberated into the blood or urine may be detected by using the anti-dlk monoclonal antibody, liver cancer may be detected simply by blood test or urine test utilizing the extracellular domain of dlk as a tumor marker. Still further, a novel therapeutic drug for cancer which has a high anticancer activity is described. The therapeutic drug for cancer is especially effective for therapy of liver cancer.

Brief Description of the Drawings

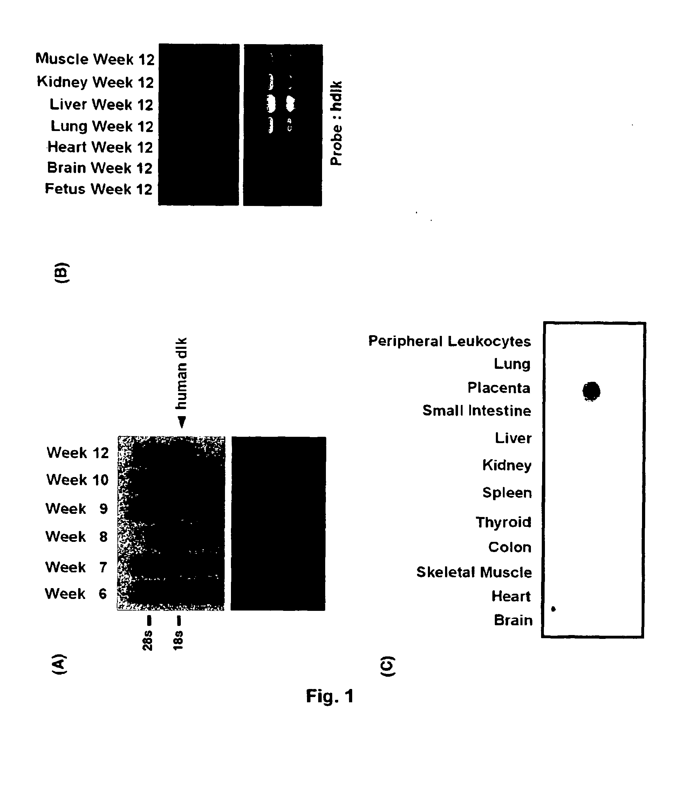

Fig. 1 are photographs showing the results of Northern blot, which indicate the expression of Dlk gene in human fetal and adult tissues. (A) Dlk gene expression in fetal liver during the 6th to 12th week of pregnancy; (B) Dlk gene expression in fetal tissues; (C) Dlk gene expression in adult tissues.

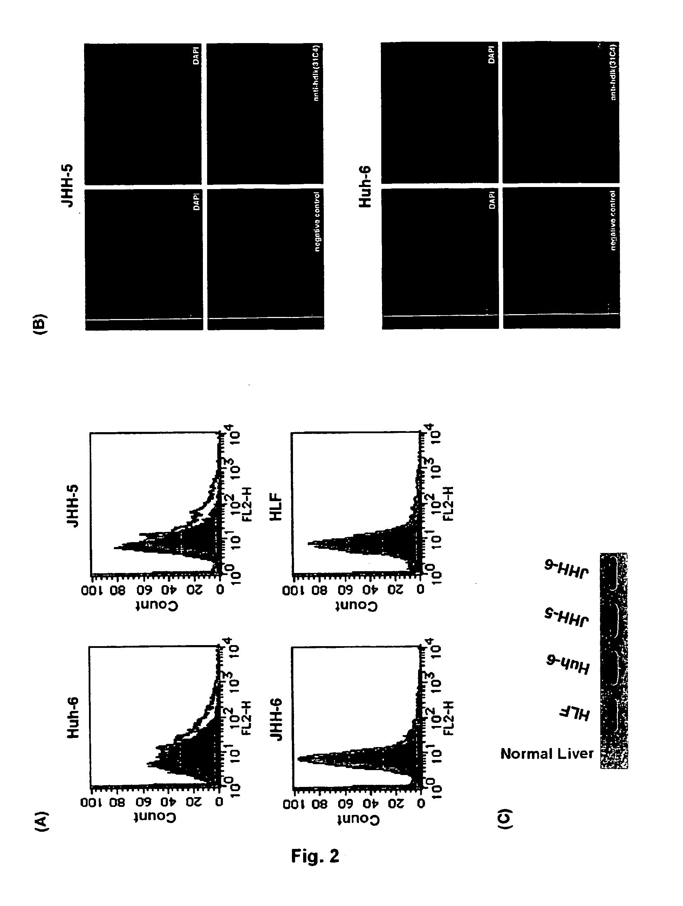

Fig. 2 shows the results of analysis for Dlk expression in the cells of cell lines derived from human liver cancer. (A) Results of FACS analysis; (b) Results of immunofluorescent staining; (C) Results of RT-PCR analysis.

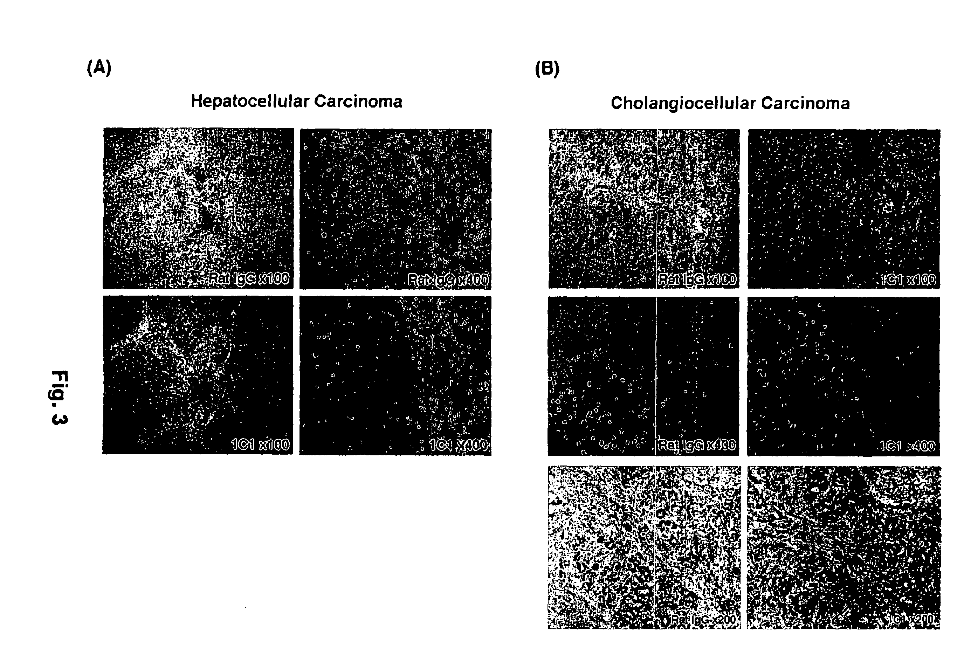

Fig. 3 are photographs showing Dlk expression in human liver cancers. (A) hepatocellular carcinoma; (B) cholangiocellular carcinoma.

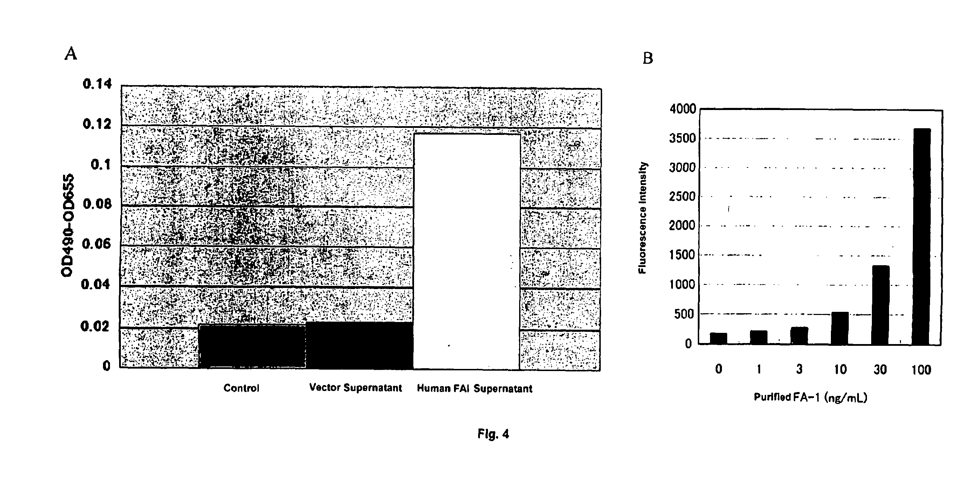

Fig. 4 (A) is graph showing the detection of human FA1 using anti-human Dlk monoclonal antibodies, and showing the results of the detection and confirmation by ELISA. (B) is a graph showing the detection of purified human FA1 using anti-human Dlk monoclonal antibodies, and showing the results of the detection and confirmation by ELISA using a chemiluminescent substrate (QuantaBlu (trademark) Fluorogenic Peroxidase Substrates: PIERCE).

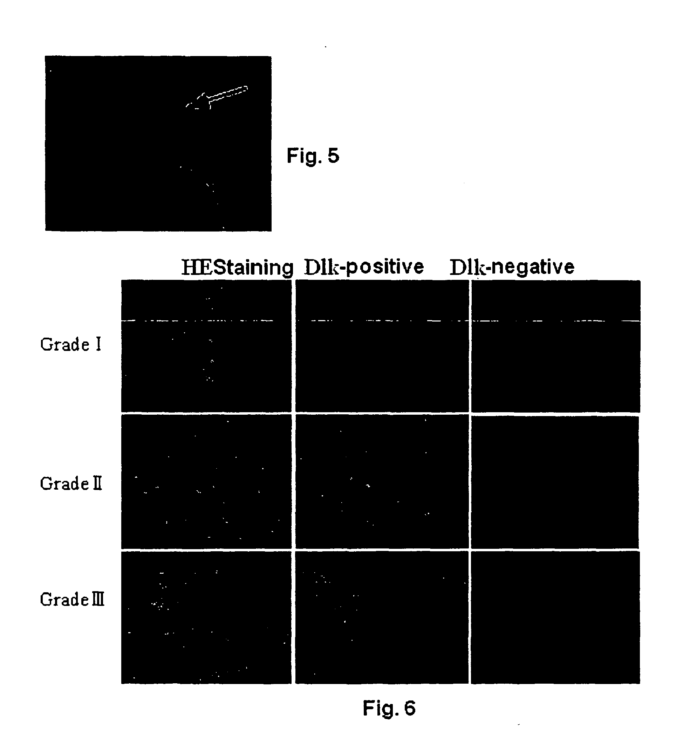

Fig. 5 shows the results of the immunostaining of human Dlk in a tissue (64 years old) based on the standard staining of Dlk. The arrow indicates the Dlk-positive area used as the standard.

Fig. 6 shows a stained image of hepatocellular carcinoma. Left: hematoxylin eosin (HE) staining; Center: immunostaining of human Dlk-positive; Right: Immunostaining of Human; Grade I: HE, Dlk-positive (48 years old, male), Dlk-negative (39 years old, male); Grade II: HE, Dlk-positive (68 years old, male), Dlk-negative (36 years old, male); Grade III: HE, Dlk-positive (63 years old, male), Dlk-negative (43 years old, male).

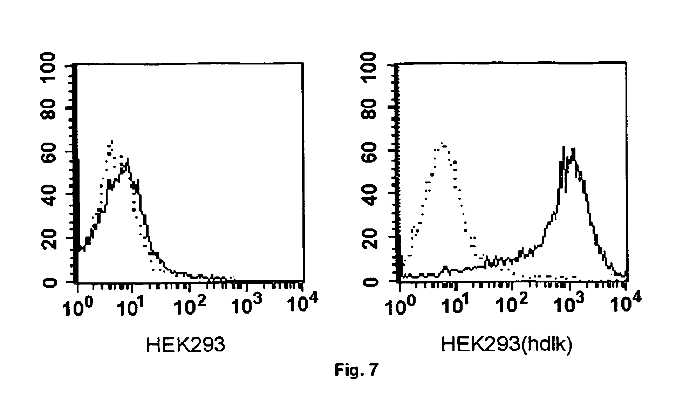

Fig. 7 shows Dlk expression of HEK293 cells and HEK293(hdlk) cells by FACS analysis. Dotted line: control IgG antibody; Solid line: anti-human Dlk monoclonal antibody.

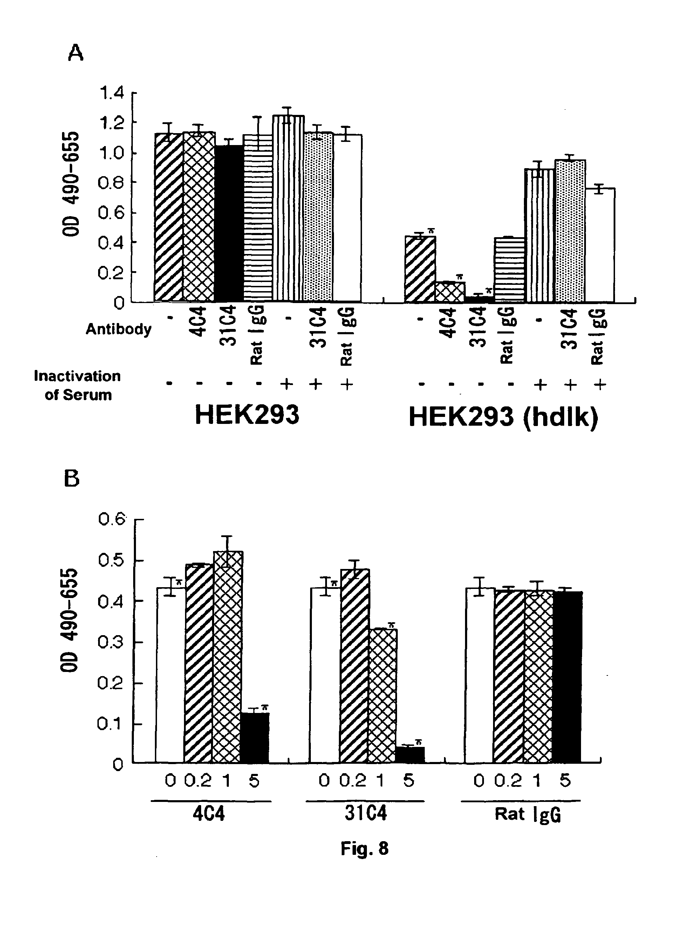

Fig. 8 shows CDC activities by anti-human Dlk monoclonal antibodies. (A) To HEK293 cells and HEK293(hdlk) cells, the antibodies were respectively added to 5 $\mu$g/ml and normal human serum was added to 25%, and the cells were cultured for three days. CDC activities were measured by MTT assay, and the measured activities are indicated as mean $\pm$ standard error. The absorbance values were significant with respect to that of the system in which no antibody was added (*: p<0.01, n=3, Student's $t$ test); (B) To HEK293(hdlk) cells, the antibodies were respectively added and normal human serum was added to 25%, and the cells were cultured for three days. CDC activities were measured by MTT assay, and the measured activities are indicated as mean $\pm$ standard error. The absorbance values were significant with respect to that of the system in which no antibody was added (*: p<0.01, n=3, Student's $t$ test)

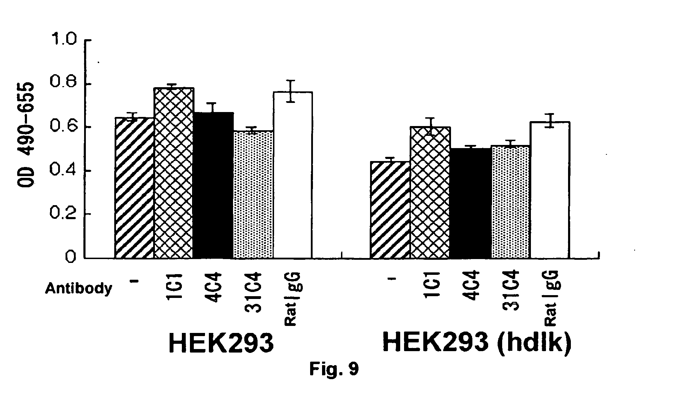

Fig. 9 shows ADCC activities by anti-human Dlk monoclonal antibodies. To HEK293 cells and HEK293(hdlk) cells, the antibodies were respectively added to 5 $\mu$g/ml and mononuclear cells in peripheral blood from a healthy individual was added to 25%, and the cells were cultured for three days. ADCC activities were measured by MTT assay, and the measured activities are indicated as mean $\pm$ standard error. The effector:target ratio was 10:1.

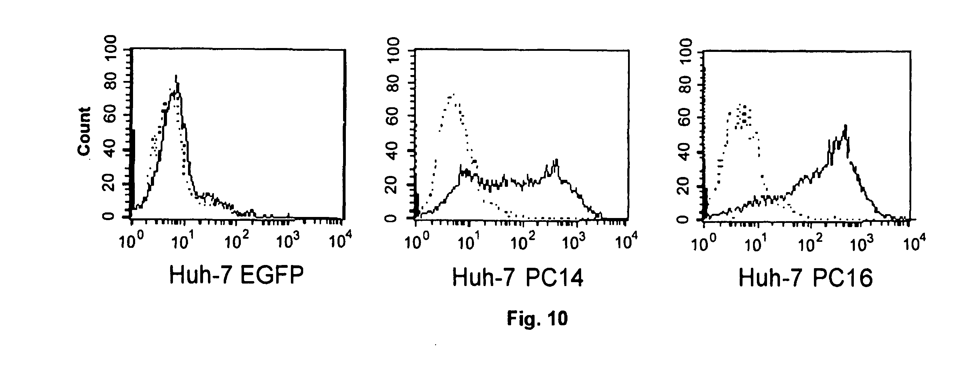

Fig. 10 shows Dlk expression of Huh-7 EGFP cells and Huh-7(hdlk) cells by FACS analysis. Dotted line: control IgG antibody; Solid line: anti-human Dlk monoclonal antibody.

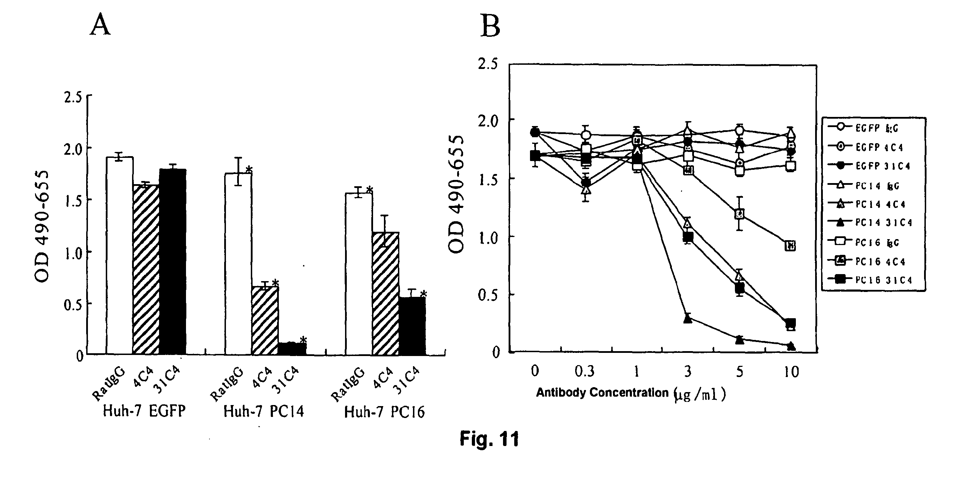

Fig. 11 shows CDC activities by anti-human Dlk monoclonal antibodies. (A) To Huh-7 EGFP cells and Huh-7(hdlk) cells (clones PC14 and PC16), the antibodies were respectively added to 5 $\mu$g/ml and normal rat complement-containing serum was added to 25%, and the cells were cultured for three days. CDC activities were measured by MTT assay, and the measured activities are indicated as mean $\pm$ standard error. The absorbance values were significant with respect to that of the system in which no antibody was added (*: p<0.01, n=3, Student's $t$ test); (B) To Huh-7(hdlk) cells (clones PC14 and PC16), the antibody was added to a level of 0, 0.3, 1, 3, 5 or 10 $\mu$g/ml, and normal rat complement-containing serum was added to 25%, and the cells were cultured for three days. CDC activities were measured by MTT assay, and the measured activities are indicated as mean $\pm$ standard error.

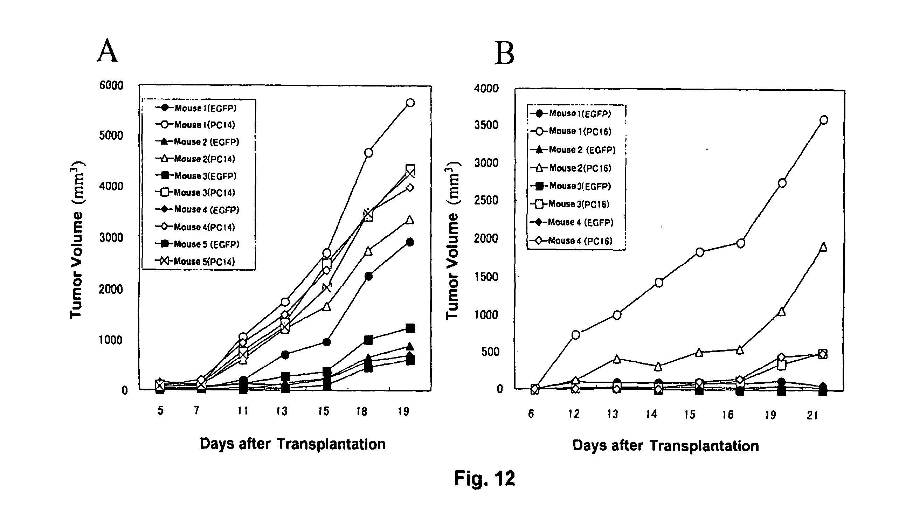

Fig. 12 is a graph showing enhancing effect of tumor-forming ability by expression of human Dlk gene. (A) Tumor formation by Huh-7 EGFP cells or by Huh-7(hdlk) cells (clone PC14) in subcutaneous areas of nude mice. Tumor volumes ($mm^3$) at 19 days from the transplantation are shown; (B) Tumor formation by Huh-7 EGFP cells or by Huh-7(hdlk) cells (clone PC16) in subcutaneous areas of nude mice. Tumor volumes ($mm^3$) at 21 days from the transplantation are shown. Best Mode for Carrying Out the Invention

As will be concretely described in Examples below, the present inventors discovered that dlk is expressed in adults on the surfaces of liver cancer cells with high specificity, and that the detection of liver cancer cells may be attained by using the dlk antigen on the cell surfaces as a tumor marker or by measuring the mRNA of dlk gene. The present invention is based on this discovery. In the present description and claims, the term "measurement" includes detection, quantification and semi-quantification.

Dlk *per se* is known and the cDNA encoding dlk has been cloned. The nucleotide sequence thereof and the amino acid sequence encoded thereby are also known. For example, the sequence of human dlk is described in GenBank Accession Nos. U15979, NM_003836 and so on. The sequence of rat dlk is described in GenBank Accession Nos. AB046763 and D84336. The sequence of bovine dlk is described in GenBank Accession No. AB009278. The cDNA sequence of human dlk as well as the amino acid sequence encoded thereby are shown in SEQ ID NOs: 1 and 2 of SEQUENCE LISTING. Further, as described in GenBank Accession No. NM_003836, a plurality of variants having a SNP(s) are known, and needless to say, these variants are included in dlk. In the amino acid of SEQ ID NO: 2, the extracellular domain is the region from 24aa to 304aa.

Since dlk is expressed on the surfaces of liver cancer cells, liver cancer cells may be detected utilizing it as a tumor marker antigen. Liver cancer cells include hepatocellular carcinoma cells and cholangiocellular carcinoma cells, and as will be concretely described in Examples below, it was confirmed that dlk is expressed on the cell surfaces of both of these carcinoma cells. The method *per se* for measuring the tumor marker antigen on cell surfaces is well-known, and may be attained by various methods utilizing the antigen-antibody reaction between the tumor marker antigen and an antibody which undergoes antigen-antibody reaction therewith. As the antibody to be used, a monoclonal antibody having a high and uniform specificity is preferred. An anti-mouse dlk monoclonal antibody is known (Non-patent Literature 11). Further, as will be concretely described, the present inventors succeeded in the preparation of anti-human dlk monoclonal antibodies. That is, a hybridoma which produces an anti-human dlk monoclonal antibody may be established by inserting a human dlk cDNA into an expression vector for mammalian cells, preparing a cell line which expresses dlk on cell surfaces by introducing the obtained recombinant vector into cells of a cell line, and establishing a hybridoma using the cells of the cell line as an immunogen by the well-known method by Kohler and Milstein. Alternatively, as described above, since the amino acid sequence of the extracellular domain of dlk and the nucleotide sequence of the cDNA encoding it are known, the extracellular domain of dlk or a part thereof may easily be prepared by a genetic engineering method or by a peptide-synthesis method. An anti-dlk monoclonal antibody may also be prepared by the well-known method using as an immunogen the prepared extracellular domain of dlk or a part thereof as it is, or after conjugating it to a carrier such as keyhole limpet hemocyanin (KLH) or bovine serum albumin (BSA). An antibody fragment having antigen-binding property, such as Fab fragment or $F(ab')_2$ fragment of the antibody may also be used.

Since methods for measuring the cells expressing an antigen on their cell surfaces using an antibody or an antigen-binding fragment thereof to the antigen (in the case of the present invention, dlk) expressing on the cell surfaces are well-known, liver cancer cells in a sample may be measured by well-known methods using an anti-dlk antibody. The measurement methods include immunostaining, sandwich methods such as ELISA, agglutination methods such as latex agglutination method, and competitive methods. Any of these methods is well-known, and may be carried out easily according to a conventional method if the antibody to be used is obtained. Preferred methods by which detection of liver cancer cells may be effectively carried out according to the present invention include the methods utilizing a magnetic cell sorter (MACS) or flow cytometer, especially fluorescence activated cell sorter (FACS). MACS is a system for separating the desired cells by labeling the cells with ultra fine particles on which an antibody to the cell surface antigen is immobilized, and passing the resulting cells through a column set in a strong magnetic field. By MACS, since highly pure cells may be obtained with a high recovery rate, and since a large number of cells may be effectively separated maintaining the functions and growing ability of the cells, MACS is preferred when the properties of the detected liver cancer cells are further investigated. FACS is an apparatus for separating the cells by labeling the cells with a fluorescence-labeled antibody, irradiating the cell flow emitted from a nozzle with a laser beam, analyzing the generated dispersed light and fluorescence, electrically charging droplets each containing one cell therein, and separating the droplets in a high electric field. Because of the same reason as MACS, FACS is also preferred to be used in the method of the present invention. Both MACS and FACS are well-known in the art and apparatuses therefor are commercially available, so that they may be easily carried out if the antibody to be used is obtained.

The sample to be subjected to the method for detecting dlk antigen on cell surfaces is a sample which may contain liver cancer cells, and usually is a biopsy sample of the liver. The biopsy sample may be a tissue section (in case of immunostaining) or may be a cell suspension obtained by treating the liver tissue with a protease such as collagenase or trypsin.

On the other hand, it has been proved that the extracellular domain of Dlk which is a membrane protein is cleaved off to yield a soluble molecule known as FA1 (Non-patent Literature 10). As described in Examples below, the anti-human dlk monoclonal antibodies prepared by the present inventors undergo antigen-antibody reaction also with FA1. Therefore, by immunoassaying FA1 in the blood using an anti-dlk antibody, especially anti-dlk monoclonal antibody, diagnosis of liver cancer may be attained using a blood sample (serum, plasma, whole blood and the like) or urine sample. Immunoassay *per se* may easily be carried out by the conventional methods described above. For example, in cases where the immunoassay is carried out according to sandwich ELISA, an anti-Dlk antibody or an antigen-binding fragment thereof as a primary antibody is immobilized on a solid phase; the immobilized primary antibody is reacted with a sample; the resultant is reacted, after washing, with a secondary antibody which undergoes antigen-antibody reaction with Dlk; and, after washing, the secondary antibody bound to the solid phase is measured. By labeling the secondary antibody with an enzyme, fluorescent substance, radioactive substance, biotin or the like, the secondary antibody bound to the solid phase may be measured. The FA1 in a test sample may be quantified by subjecting a plurality of samples each containing a known level of FA1 to the above-described immunoassay; preparing a calibration curve based on the relationship between the each of the measured amounts of the label and each of the amounts of FA1 in standard samples; and applying the measurement result of a test sample containing an unknown amount of FA1 to the calibration curve. As will be concretely described in Examples below, a detection sensitivity of as high as 1 ng/mL or less may be attained by using a luminescent substance (fluorogenic peroxidase substrate: PIERCE). In agglutination method, an anti-Dlk antibody or an antigen-binding fragment thereof is immobilized on particles such as latex, the resulting particles are reacted with a sample, and absorbance is measured. The absorbance is measured by the above-described method for each of a plurality of standard samples each containing a known level of FA1 and a calibration curve is prepared based on the measurement results. The FA 1 in a test sample containing an unknown level of FA1 may be quantified by applying the measurement result of the sample to the calibration curve.

In the above-described method for measuring the dlk antigen on cell surfaces utilizing antigen-antibody reaction, in cases where the dlk antigen on human cells is to be measured, it is preferred, needless to say, to use an anti-human dlk monoclonal antibody or an antigen-binding fragment thereof.

As described above, since anti-dlk antibodies, preferably anti-dlk monoclonal antibodies may be used for the detection of liver cancer, they have the use as a diagnostic for liver cancer.

Expression of dlk gene may also be determined by measuring the mRNA of dlk in the cells. The measurement of mRNA in the cells may be carried out by conventional methods. That is, for example, as described in Examples below, it may be carried out by Northern blot; or by carrying out reverse transcription PCR (RT-PCR), electrophoresing the PCR product, and subjecting the resulting electrophoretic bands to Southern blot. Alternatively, it may be measured by directly amplifying the mRNA by NASBA or the like, electrophoresing the amplified product, and subjecting the resultant to Northern blot. All of these methods *per se* are conventional methods and the required reagents kits and apparatuses are commercially available. Further, since the cDNA sequence of Dlk is known, the probes and primers required in these methods may easily be designed, and examples of these are also described concretely in Examples below. Therefore, measurement of mRNA encoding Dlk protein may easily be carried out by those skilled in the art. Although each of the probes and primers used in the detection or amplification of the mRNA (or the cDNA obtained by using the mRNA as a template) of Dlk preferably has a sequence complementary to either chain of the mRNA or cDNA of Dlk, it is possible to use a probe or primer having a mismatch(es) in the number of not more than 10%, preferably not more than 5% of its size. By using a primer having such a mismatch(es), a desired restriction site may be given to the amplification product. Such a restriction site may be convenient in inserting the amplification product into a vector. The size of the probe or primer (the size of the region which hybridizes with the mRNA or cDNA of Dlk) is not restricted, and is not less than 15 bases, preferably not less than 20 bases as in the conventional methods. The upper limit of the size is not restricted and the size is usually not more than 50 bases, preferably not more than 40 bases. In case of a probe, one having a size of the full length or less is appropriate. As long as a nucleic acid fragment contains a region which hybridizes with a region in the mRNA or cDNA of Dlk to be measured and can be used as a primer or probe, a non-complementary sequence may be attached to an end of the nucleic acid fragment. Such an additional sequence may be used for the binding with a tag or another nucleic acid. Also described herein is a nucleic acid for detecting liver cancer, which hybridizes with the mRNA or cDNA of Dlk, such as these probes and primers.

As described above, the therapeutic drug for cancer contains as an effective ingredient an antibody which undergoes antigen-antibody reaction with Dlk expressing on cancer cell surfaces. Among the above-described anti-Dlk antibodies, anti-Dlk antibodies each of which exerts anticancer activity against the cancer cells expressing Dlk on cell surfaces may be used as the antibody which undergoes antigen-antibody reaction with Dlk, and monoclonal antibodies having a high and uniform specificity are preferred. The anti-dlk monoclonal antibody which exerts anticancer activity against the cancer cells expressing Dlk on their surfaces may be screened by the MTT assay using the cells of a Dlk-expressing cell line, which assay is concretely described in Examples below. Since two types of anti-human Dlk monoclonal antibodies among the obtained three types of anti-human Dlk monoclonal antibodies exerted anticancer activity in MTT assay, an anti-dlk monoclonal antibody which exerts anticancer agent against the cancer cells expressing Dlk on their surfaces may be obtained with reproducibility by the screening by MTT assay.

Although the antibody may be one derived from an animal species different from the animal species to which the therapeutic drug is to be administered, the antibody is preferably one at least whose constant region is the same constant region (Fc) of the antibody of the same animal species to which the drug is to be administered. For example, in case of a therapeutic drug to be administered to human, a chimeric antibody or humanized antibody whose constant region at least is derived from human may preferably be employed. By using a chimeric antibody or humanized antibody, the antigenicity of the antibody can be decreased, and occurrence of antibody-antigen reaction when the antibody is administered is decreased. In addition, in cases where the antibody is administered to human, by employing an antibody whose constant region is derived from human, it is thought that ADCC activity is increased. This is because that, it is necessary that Fc of the antibody be bound to the Fc receptor of the effector cells in order that ADCC may occur, and so it is advantageous that the Fc fit the Fc receptor on the effector cells of the animal species. A chimeric antibody is an antibody obtained by immunizing a mouse with an antigen, separating the region of the gene of the monoclonal antibody obtained, which region encodes the variable region (V region) of the antibody, that binds to the antigen, ligating the separated region to the gene encoding the constant region (C region) of an antibody derived from a human myeloma cell to prepare a chimeric gene, and expressing the obtained chimeric gene in a host cell. The preparation method thereof is well-known, and a number of chimeric antibodies are commercially available. A humanized antibody is an antibody encoded by a gene whose region encoding the antigen-binding site (CDR, complementarity-determining region) alone is transplanted to a human antibody gene, and is an antibody in which the region derived from mouse is still smaller than in chimeric antibodies. Humanized antibodies and their preparation methods are well-known, and a number of humanized antibodies are commercially available in recent years.

As will be concretely described in Examples below, an anti-Dlk antibody exerts anticancer activity at least in the presence of complement. Since complement is contained in the blood of a patient, the anti-Dlk antibody functions as a therapeutic drug for cancer as it is. In the Examples below, although ADCC activity of the anti-human Dlk monoclonal antibody against the cells of human liver cancer cell line was not observed, this is presumably because that the Fc regions of the antibodies were derived from rat. Since CDC activity is observed, it is thought that ADCC will also be exerted if the Fc region is replaced with that of human. Although the anti-Dlk antibody may be used as it is, by conjugating the antibody with a toxin such as ricin or other anticancer agent, the so called missile therapy may also be attained.

The cancers cured by the therapeutic drug for cancer are the cancers in which Dlk is expressed on the surfaces of the cancer cells.

Examples of the cancers include liver cancer such as hepatocellular carcinoma and cholangiocellular carcinoma; lung small cell carcinoma; and type 1 neurofibromatosis. Among these cancers, liver cancer such as hepatocellular carcinoma and cholangiocellular carcinoma is most preferred.

The therapeutic drug for cancer is preferably administered through a parenteral route such as injection to the affected part, intravenous injection, intramuscular injection or the like. The dosage per day per adult is usually about 0.001 to 100 mg, preferably about 0.01 to 50 mg, still more preferably about 0.1 to 5 mg in terms of the weight of the antibody per 1 kg of body weight. The formulation may be one containing the antibody dissolved in physiological buffer, and one or more additives generally used in field of the formulation of pharmaceuticals may be added.

The present invention will now be described more concretely by way of Examples. It should be noted, however, the present invention is not restricted to the Examples below.

Examples

Example 1 Detection of Liver Cancer

1. Materials and Methods

(1) Isolation of Full Length Human dlk cDNA and Construction of Expression Vector

PCR primers were designed based on the information of gene sequence of human Dlk (GenBank accession No. U15979). The sequences of the prepared primers were as follows:

Forward Primer: 5'-cgcgtccgcaaccagaagccc-3'
Reverse Primer 5'-aagcttgatctcctcgtcgccggcc-3'

To the reverse primer, a restriction site of *Hind* III was added. PCR was performed using these primers and cDNAs synthesized from the total RNAs (TAKARA) prepared from the human liver of embryonic week 10. Then the PCR product was developed in agarose gel electrophoresis, and the desired band was extracted, followed by cloning the amplification product into pCRII vector (Invitrogen) (pCRII-hdlk). Existence of the cloned cDNA of human Dlk was confirmed by sequencing.

In the construction of the expression vector, to attach a Flag tag to the C-terminal of human Dlk, firstly, oligonucleotides encoding the Flag tag sequence were inserted into the *Hind*III/*Sal*I site of pBluescript II SK(+) vector (STRATAGENE) (Sequences: forward side: 5'-agcttgactacaaggacgacgatgacaagtgag-3', reverse side: 5'-tcgactcacttgtcatcgtcgtccttgtagtca-3') (pBS-Flag). Then an *Eco*RI/*Hind*III fragment was cleaved out from pCRII-hdlk and was inserted into the *Eco*RI/*Hind*III site of the pBS-Flag vector (pBS-hdlk-Flag). An *Eco*RI/*Sal*I fragment was cleaved out from pBS-hdlk-Flag and was inserted into the *Eco*RI/*Xho*I sites of pcDNA3.1 vector (Invitrogen) and pMIG vector, respectively (pcDNA-hdlk-Flag and pMIG-hdlk-Flag, respectively).

For constructing an expression vector which expresses human FA1, the following primers were designed and synthesized:

Forward Primer: 5'-cgcgtccgcaaccagaagccc-3'
Reverse Primer: 5'-ctcgaggtgctccggctgctgcaccggc-3'

In this case, the restriction site of *Xho*I was added to the reverse primer. PCR was performed using these primers and cDNA of human dlk as a template, and the obtained human FA1 cDNA was cloned into pCRII vector (Invitrogen) (pCRII-hFA1). Existence of the cloned human FA1 cDNA was confirmed by sequencing.

The *Eco*RI/*Xho*I fragment containing the human FA1 cDNA was cleaved out from pCRII-hFA1, and was inserted into the *Eco*RI/*Xho*I site of pcDNA4/Myc-His vector (Invitrogen) (pcDNA4-hFA1). This expression vector contains Myc tag and His tag sequences at the C-terminal, and human FA1 is expressed in the form of a fusion protein with Myc tag and His tag.

(2) Cell Line Derived from Human Liver Cancer

The cell lines derived from human liver cancer were JHH-6, HLF, JHH-5 and Huh-6, and all of them were furnished by Japan Health Sciences Foundation.

(3) Introduction of Gene into Cultured Cells

Introduction of the gene into cultured cells was carried out using LipofectAMINE-plus reagent (GIBCO BRL), in accordance with the protocol described in the attached instructions.

(4) RT-PCR

RNAs were extracted from the cells of the human cancer liver-derived cell lines using Trizol reagent (Nippon Gene). cDNAs were synthesized from the extracted RNAs using First-strand cDNA synthesis kit (Amersham Pharmacia Biotech), and expression of human Dlk was analyzed by PCR. The used primers were as follows:

Forward Primer: 5'-agagctcaacaagaaaacc-3'
Reverse Primer: 5'-gcgtatagtaagctctgagg-3'

(5) Northern Blot Analysis

Fetal tissue total RNAs (TAKARA) and the total RNAs extracted from the cells using Trizol reagent (Nippon Gene), in an amount of 10 $\mu$g each, were electrophoresed on formaldehyde-denatured gel. After transferring the bands to a Nylon membrane, hybridization with a DIG-labeled cDNA probe was performed. Detection of the probe was carried out by chemiluminescence using CDP-star as a substrate.

(6) Preparation of Anti-human Dlk Monoclonal Antibodies

The above-described retrovirus vector (pMIG-hdlk-Flag) in which the human dlk gene was incorporated was introduced into BOSC23 cells (Pear, W.S. et al. (1993) Proc. Natl. Acad. Sci. USA 90, 8392-8396) that are packaging cells, to prepare a retrovirus having the human dlk gene. Cells of cell line 7E2-C which we previously established from the fetal liver of a transgenic mouse producing temperature-sensitive SV40 large T antigen (Yanai,N. et al. (1991) Exp. Cell Res. 197, 50-56) were infected with the produced retrovirus to obtain a cell line 7E2-C(hdlk) consistently expressing human Dlk.

The above-described expression vector pcDNA-hdlk-Flag was introduced into HEK293 cells (obtained from Laboratory of Cell Growth and Differentiation, Institute of Molecular and Cellular Biosciences, The University of Tokyo), and after selection with an antibiotic G418 (geneticin, GIBCO BRL), a cell line HEK293(hdlk) which stably expresses human Dlk was established.

Rats were immunized with cells of the above-described two types of cell lines as antigens, respectively, and hybridoma clones each of which produces an anti-human Dlk monoclonal antibody were prepared by a conventional method. The cells of each of these clones were intraperitoneally administered to BALB/c nude mice at a dose of 3 x 10$^6$ cells, respectively, which nude mice preliminarily (7 days before) received 2,6,10,14-tetramethylpentadecane (pristane), and two weeks later, ascites were recovered from the mice. The anti-human Dlk monoclonal antibodies each of which was produced by each hybridoma were obtained by subjecting the ascites to caprylic acid precipitation and to purification with a protein G column.

(7) Cell ELISA

The cells of the above-described 7E2-C(hdlk) cell line were placed in a gelatin-coated 96-well culture plate (Coming) in an amount of 7.5 x 10³ cells/well, and cultured at 37°C for 2 days. After washing the plate with ice-cold PBS, the cells were fixed with 4% paraformaldehyde solution and treated with 0.2% Triton-X-100 (trademark) solution to prepare a plate for cell ELISA. Thereafter, ELISA was performed according to a conventional method. More particularly, ELISA was performed as follows. First, blocking with 1% BSA-PBS solution was carried out at room temperature for 2 hours. The hybridoma supernatant was then added to the plate, and the resulting mixture was allowed to react at room temperature for 1 hour, followed by washing the plate 3 times with 0.1 % Tween 20 (trademark)-PBS solution. As a secondary antibody, biotinylated anti-rat IgG (produced by VECTOR) 100-fold diluted with 0.1% Tween 20-PBS solution was used. After allowing reaction at room temperature for 1 hour, the plate was washed 3 times with 0.1% Tween 20-PBS solution. Then the resultant was reacted with horseradish peroxidase-streptavidin (produced by VECTOR) 1000-fold diluted with 0.1 % Tween 20-PBS solution at room temperature for 1 hour, and then the plate was washed 3 times with 0.1 % Tween 20-PBS solution. A solution of TMB (3,3',5,5'-tetramethylbenzidine, produced by SIGMA) as a substrate was added to allow coloring reaction, and 1M sulfuric acid was added to stop the reaction. The absorbance was measured with Microplate reader Model 550(BIO-RAD).

(8) Immunohistochemical staining

Paraffin sections of normal human tissue and liver cancer tissue (Bio Chain, Hepatocellular carcinoma; catalog No.:T2235149-4, lot No.:A607070, Cholangiocellular carcinoma; catalog No.:T2235149-2, lot No.:A603549) were deparaffinized and heat-treated in 10 mM sodium citrate solution for 10 minutes. The resulting sections were used for staining using the anti-human Dlk monoclonal antibodies. After performing coloring reaction with DAB (3,3'-diaminobenzidine) as a substrate, nuclear stain with hematoxylin was performed as a counter staining. More concretely, these operations were carried out as follows. The sections fixed with 4% paraformaldehyde and embedded in paraffin were deparaffinized and heat-treated in 10 mM sodium citrate solution for 10 minutes. The resulting sections were treated with methanol to which aqueous hydrogen peroxide solution was added to a final concentration of 0.3% at room temperature for 20 minutes to remove endogenous peroxidase activity. After washing the resulting sections twice with PBS at room temperature for 5 minutes/wash, blocking was performed with Block Ace (DAINIPPON PHARMACEUTICAL) for 30 minutes to block the non-specific binding sites in the tissues. Then the resultant sections were reacted with a solution of anti-human dlk monoclonal antibody clone 1C1 (final concentration of $0.25\mu$ g/ml) diluted with 10-fold diluted Block Ace at room temperature for 1 hour, and after washing 3 times with PBS for 5 minutes/wash, the resulting sections were reacted with biotinylated anti-rat IgG antibody 100-fold diluted with 10-fold diluted Block Ace at room temperature for 1 hour. After washing three times with PBS for 5 minutes/wash, ABC complex prepared by mixing the reagents contained in ABC kit in accordance with the instructions was reacted with the resulting sections at room temperature for 30 minutes. After washing three times with PBS for 5 minutes/wash, coloring was carried out with peroxidase substrate (0.02% DAB, 0.03% aqueous hydrogen peroxide solution, 50 mM Tris-HCl pH 7.5). After confirming the coloring, the sections were washed with water for 10 minutes, and the nuclei were stained with Mayer-hematoxylin solution (WAKO). Thereafter, the sections were dehydrated with alcohol, xylene-cleared, and embedded in Entellan New (MERK JAPAN).

On the other hand, paraffin sections of human hepatocellular carcinoma (CYBRDI, Hepatocellular carcinoma; catalogue No.: CS03-01, lot No.: CS03-01-001-012 (23 patients, 63 sections), CS03-01-002 (63 patients, 63 sections)) were deparaffinized, hydrophilized, and treated with 10 mM citrate buffer (pH6.0) in an autoclave (121°C, 5 minutes). The resulting sections were treated with methanol to which aqueous hydrogen peroxide solution was added to a final concentration of 0.3% at room temperature for 20 minutes to remove endogenous peroxidase activity. After washing the resulting sections 3 times with PBS at room temperature for 5 minutes/wash, blocking was performed with 1.5% goat serum in PBS for 30 minutes to block the non-specific binding sites in the tissues. Then the resultant sections were reacted with a solution of anti-human dlk monoclonal antibody clone 1C1 (final concentration of 0.25 μg/ml) diluted with 1.5% goat serum in PBS at 4°C overnight, and after washing 3 times with PBS for 5 minutes/wash, the resulting sections were reacted with biotinylated anti-rat IgG antibody (VECTOR) 100-fold diluted with 1.5% goat serum in PBS at room temperature for 2 hours. After washing three times with PBS for 5 minutes/wash, ABC complex was reacted with the resulting sections at room temperature for 30 minutes. After washing three times with PBS for 5 minutes/wash, coloring was carried out with peroxidase substrate (0.02% DAB, 0.03% aqueous hydrogen peroxide solution, 50 mM Tris-HCl pH 7.5). After confirming the coloring, the sections were washed with purified water for 10 minutes, and the nuclei were stained with Mayer-hematoxylin solution (WAKO). Thereafter, the sections were dehydrated with alcohol, xylene-cleared, and embedded in Entellan New (MERK JAPAN).

(9) FACS Analysis

Cells were peeled off from the culture plate by trypsin treatment, and a cell suspension (cell population density: $5 \times 10^6$ cells/ml) was prepared. Then 0.5 $\mu$g of the each anti-human Dlk monoclonal antibody and 100 $\mu$L of the cell suspension were reacted at 4°C for 30 minutes. After washing the cells with PBS, the cells were reacted with biotinylated anti-rat IgG (VECTOR) (0.5 $\mu$g) and then again washed with PBS. After reacting (4°C, 30 minutes) the resulting cells with streptavidin-FITC (Pharmingen) or streptavidin-PE (Pharmingen) (0.5 $\mu$g), the cells were analyzed with FACSCalibur (BECTON DICKINSON).

(10) Detection of Human FA1 by Anti-human dlk Monoclonal Antibodies

Human FA1-expressing vector was introduced into 7E2-C cells, and the culture supernatant 3 days after thereof, or hFA1 purified from the culture supernatant by His Trap HP Kit (Amersham Bioscience) (hFA1 concentration: 30 $\mu$g/ml) was used as the detection sample. Sandwich ELISA using clone 31C4 as the capture antibody and biotinylated clone 4C4 as the detection antibody was employed for the detection. The biotinylation of the detection antibody was carried out using ECL™ Protein Biotinylation Module(Amersham Bioscience). More concretely, this sandwich ELISA was carried out as follows. First, the capture antibody clone 31 C4 was diluted with PBS to 10 $\mu$g/ml, and added to a 96-well plate in an amount of 100 $\mu$l/well. After leaving the plate to stand at room temperature overnight, the plate was washed 3 times with PBS, and blocking with 2% skim milk in PBS (hereinafter referred to as "2% MPBS") was carried out at room temperature for 2 hours. Then the culture supernatant containing hFA1 or hFA1 diluted with 2% MPBS to the respective concentration was added, and the plate was left to stand at room temperature for 1 hour. After washing the plate 3 times with PBS, biotinylated clone 4C4 as the detection antibody, diluted with 2% MPBS to 1 $\mu$g/ml, was added. After allowing reaction at room temperature for 1 hour, the plate was washed 3 times with 0.1 % Tween 20 (trademark)-PBS solution. As the secondary antibody, biotinylated anti-rat IgG (VECTOR) 100-fold diluted with 2% MPBS solution was used. After allowing reaction at room temperature for 1 hour, the plate was washed three times with 0.1% Tween 20-PBS solution. After allowing reaction with horseradish peroxidase-streptavidin (produced by VECTOR) 1000-fold diluted with 2% MPBS at room temperature for 1 hour, the plate was washed 3 times with 0.1 % Tween 20-PBS solution. Coloring reaction was carried out by adding TMB (3,3',5,5'-tetramethylbenzidine: produced by SIGMA), and the reaction was stopped by adding 1 M sulfuric acid. Absorbance was measured with Microplate reader Model 550(BIO-RAD). Fluorescence reaction was measured using QuantaBlu (trademark) Fluorogenic Peroxidase Substrates (produced by PIERCE) and Fluoroscan Ascent (produced by THERMO LABSYSTEMS) as a measuring apparatus.

2. Results

(1) Expression of Human Dlk in Human Normal Liver

The present inventors previously discovered that Dlk highly expresses in fetal hepatic cells, the expression is not observed in adult hepatic cells and that stem cells alone may be recovered from fetal liver with a high purity by using an anti-mouse Dlk monoclonal antibody in combination with MACS (magnetic beads cell sorting) (Non-patent Literature 7, Patent Literature 1). Thus, whether Dlk shows the similar expression pattern in human or not was first investigated. By Northern blot analysis of total RNA sample (TAKARA) from human fetal liver, expression of human Dlk was observed in fetal liver during the 6th to 12th week of pregnancy (Fig. 1A). Expression of human Dlk was also investigated in various fetal organs at 12th week of pregnancy. As a result, Dlk was expressed also in the kidney and skeletal muscle in addition to the liver (Fig. 1B). In contrast, in adult tissues, expression of Dlk was not detected except for placenta (Fig. 1C) as previously reported (Non-patent Literature 1). However, it was reported recently that FA1 is also expressed in pituitary gland (Larsen, J.B. et al. (1996) Lancet. 347, 191) and in adrenal gland (Jensen, C.H. et al. (1993) Hum. Reprod. 8, 635-641). Thus, it was proved that in human, although expression of Dlk in the liver is observed in fetus, it is not expressed in adult liver as in mouse.

(2) Anti-Dlk Monoclonal Antibody

To further confirm the above-described results, the present inventors first prepared anti-human Dlk monoclonal antibodies (rat IgG). Two types of human Dlk-expressing cells as antigens were established, and rats were immunized with these cells as antigens. Hybridomas were prepared according to a conventional method, and positive clones were selected by FACS analysis using the 7E2-C(hdlk) cells used as the antigen and by cell ELISA. Cloning was further carried out and three stable clones (clones 1C1, 4C4 and 31 C4) were established. By FACS analysis using the each culture supernatant of the finally established clones, it was confirmed that a monoclonal antibody which specifically reacts with human Dlk was contained in each culture supernatant.

The cells of each of these clones were intraperitoneally administered to BALB/c nude mice at a dose of 3 x $10^6$ cells, respectively, which nude mice preliminarily (7 days before) received 2,6,10,14-tetramethylpentadecane (pristane), and two weeks later, ascites were recovered from the mice. The anti-human Dlk monoclonal antibodies each of which was produced by each hybridoma were obtained by subjecting the ascites to caprylic acid precipitation and to purification with a protein G column. Each of the obtained purified monoclonal antibodies showed an activity comparable to that observed for each culture supernatant in FACS analysis.

Using the obtained anti-human Dlk monoclonal antibody clone 1C1, immunohistochemical staining of human fetal tissues was performed. In agreement with the results of Northern blot, stained areas were observed in the liver, kidney and skeletal muscle. Placenta tissue was also stained in the same manner, and strong staining was observed in syncytiotrophoblasts in villi.

(3) Expression of Human Dlk in Cell Line Derived from Human Liver Cancer

Similar to the results in mouse, although expression of human Dlk is observed in immature fetal liver cells, it is not observed in adult liver cells. The present inventors studied the possibility of expression of human Dlk in human liver cancer. First, 4 types of cell lines (JHH-6, HLF, JHH-5 and Huh-6) derived from human liver cancer were examined by FACS analysis, immunostaining and RT-PCR. FACS analysis was carried out using anti-human Dlk monoclonal antibody clone 4C4. As a result, with the undifferentiated type cell lines (JHH-6 and HLF), the shift indicating the expression of human Dlk was not observed, but with the differentiated type cell lines (JH-5 and Huh-6), the shift was observed (Fig. 2A). As a result of the immunostaining, similarly, stained areas were observed in the differentiated cell lines (Fig. 2B).

Analysis by RT-PCR was then carried out. From the total RNAs extracted from each of the cell lines, cDNAs were synthesized, and PCR was performed using the obtained cDNAs as templates. As a result, similar to the results of the FACS analysis and immunostaining, expression of human Dlk was observed in the differentiated type cell lines. However, by the RT-PCR, expression of human Dlk was also observed in the undifferentiated type cell lines even though it was weak (Fig. 2C), which was not observed in FACS analysis and immunostaining. The difference between the results with the differentiated type cell lines is thought to stem from the difference in the detection sensitivities.

(4) Expression of Human Dlk in Human Liver Cancer Tissue

The results of the analyses of expression of human dlk in cell lines derived from human liver cancer suggest the possibility that human Dlk may be expressed in liver cancer tissue. Thus, expression of human Dlk in human liver cancer tissue was examined by immunohistochemical staining using the anti-human Dlk monoclonal antibody clone 1C1. As a result, it was proved that the cancerous parts in hepatocellular carcinoma tissue and cholangiocellular carcinoma tissue were strongly stained (Fig. 3). In these cases, the normal tissue adjacent to the cancerous part was not stained at all. This indicates that Dlk is not only expressed in the fetal liver cells, but also expressed by the canceration of adult liver cells. Thus, it was suggested that Dlk may be used as a tumor marker for liver cancer.

To accurately determine the positive rate of Dlk in hepatocellular carcinoma, pathologic sections obtained from a number of hepatocellular carcinoma patients were examined by immunostaining using the anti-Dlk antibodies. Expression of human Dlk in hepatocellular carcinoma using a human tissue array was evaluated based on the No. 51 section of lot No.: CS03-01-001-012 as a standard, and those which showed a staining with an intensity comparable to or higher than the Dlk-positive area in the standard were evaluated as Dlk-positive, and those which showed a staining with less intensity than the standard was evaluated as Dlk-negative (Fig. 5). Hepatocellular carcinoma tissues from 80 patients or 118 sections were examined. In the total 118 pathologic sections, 65 sections (55%) were Dlk-positive. The positive rate per each Grade was further studied. As a result, Dlk-positive rate in the hepatocellular carcinoma of Grade I was 82% (9/11), that of Grade I-II was 100% (3/3), that of Grade II was 61% (33/54) and that of Grade III was 40% (20/50) (Table 1). Thus, it was proved that in all of the hepatocellular carcinoma from the poorly differentiated type to highly differentiated type, Dlk was positive widely. Since higher Dlk expression was observed in poorly differentiated hepatocellular carcinoma of Grades I and II, and since Dlk emerges in highly proliferative fetal hepatocytes and in oval cells in the regenerating liver in adults for which the possibility of being precancerous cells has been pointed out, the high positive rate of Dlk suggest the possibility that Dlk may be a tumor marker of hepatocellular carcinoma at an early stage. Examples of the observed images of the tissues by hematoxylin eosin (HE) staining, and of the Dlk-positive and Dlk-negative tissues by Dlk staining, which tissues belonging to different Grades, respectively, are shown in Fig. 6.

It should be noted that the original photographs of Figs. 2B, 3, 4, 5 and 6 are color photographs. Although the results may not be clear from the appended drawings (black-and-white gray scale), the above-described results are clearly shown in the original photographs.

Table 1

| Grade | Total | Dlk-positive | (%) | Dlk-negative | (%) |
|---|---|---|---|---|---|
| I | 11 | 9 | (82) | 2 | (18) |
| I - II | 3 | 3 | (100) | 0 | (0) |
| II | 54 | 33 | (61) | 21 | (39) |
| III | 50 | 20 | (40) | 30 | (60) |

(5) Detection of Human FA1 by Anti-human Dlk Monoclonal Antibodies

It has been proved that extracellular domain of Dlk is cleaved to produce a soluble molecule known as FA1. Since the anti-human Dlk monoclonal antibodies we produced recognize the extracellular domain of Dlk, it was thought that human FA1 may possibly be recognized and detected using the antibody. Thus, study was made by ELISA using culture supernatant of the 7E2-C cells transiently expressing human FA1. As a result, it was confirmed that signals were detected for the culture supernatant containing human FA1 while no signals were detected for the culture supernatant of the cells into which a control vector was introduced (Fig. 4). These results proved that the anti-human Dlk monoclonal antibody we prepared can detect human FA1.

(6) Test for Sensitivity of Detection Method of Human FA1 Using Anti-human Dlk Monoclonal Antibodies

Further, as described above in the section "(10) Detection of Human FA1 by Anti-human dlk Monoclonal Antibodies" in "1. Materials and Methods", the sensitivity of the ELISA was determined using purified human FA1 protein. As a result, by using a chemiluminescent substrate (QuantaBlu (trademark) Fluorogenic Peroxidase Substrates (produced by PIERCE) and Fluoroscan Ascent (produced by THERMO LABSYSTEMS) as a measuring apparatus, human FA1 at a level of 1 ng/ml was able to be detected (Fig. 4B, Table 2).

Table 2

| FA-1 (ng/mL) | Fluorescence Intensity |
|---|---|
| 0 | 179.85 |
| 1 | 221.55 |
| 3 | 277.1 |
| 10 | 537.9 |
| 30 | 1331.0 |
| 100 | 3685.0 |

Example 2 Anticancer Activity of Anti-Dlk Antibodies

1. Materials and Methods

"(1) Isolation of Full Length Human dlk cDNA and Construction of Expression Vector", "(2) Cell Line Derived from Human Liver Cancer", "(3) Introduction of Gene into Cultured Cells", "(4) RT-PCR" and "(5) Northern Blot Analysis" were carried out as in Example 1.

(6) Preparation of Anti-human Dlk Monoclonal Antibodies

The procedures up to the establishment of the two types Dlk-expressing cell lines 7E2-C(hdlk) and HEK293 (hdlk) were carried out as in Example 1.
To prepare an anti-human Dlk monoclonal antibody, each cell suspension of the two types of Dlk-expressing cell lines 7E2-C(hdlk) and HEK293(hdlk) was mixed with an immunization aid (Freund's complete adjuvant: WAKO PURE CHEMICALS) at a ration of 1:1, and the obtained emulsion was injected to both feet of a Wister rat of 6 week age in an amount of $1 \times 10^7$ cells/foot, thereby immunizing the animal. After booster twice, lymph nodes of the both legs were recovered, lymphocytes were prepared therefrom, and cell fusion with mouse myeloma cell line (P3X) was carried out by the polyethylene glycol method. The fused cells were incubated in a medium containing HAT (aminopterin, hypoxanthine, thymidine) in a 96-well flat-bottomed plate under 5% $CO_2$ in an incubator. After the culturing, the culture supernatants of the grown hybridomas were subjected to screening by FACS analysis and cell ELISA using 7E2-C(hdlk) cell lines, thereby selecting positive clones. These clones were further cloned to establish 3 types of hybridoma (clones 1C1, 4C4 and 31C4). These hybridomas were separately suspended in RPMI medium to a population density of $1.5 \times 10^7$ cells/ml. Each of the cell suspensions was intraperitoneally administered to BALB/c nude mice (Balb/c-nu/nuSlc) in an amount of 200 $\mu$L ($3 \times 10^6$ cells), which nude mice preliminarily received 2,6,10,14-tetramethylpentadecane (pristane) 7 days before, and ascites were recovered from the mice two weeks later. The anti-human Dlk monoclonal antibodies each of which was produced by each hybridoma were obtained by subjecting the ascites to caprylic acid precipitation and to purification with a protein G column. Each of the obtained purified monoclonal antibodies showed an activity comparable to that observed for each culture supernatant in FACS analysis.

"(7) Cell ELISA", "(8) Immunohistochemical staining" and "(9) FACS Analysis" were carried out as in Example 1.

(10) Isolation of Human Peripheral Blood Mononuclear Cells

Venous blood was collected in the presence of heparin from a healthy individual, and after being 2-fold diluted with PBS, overlaid on Lymphoprep (DAIICHI PURE CHEMICALS), followed by centrifugation at 20°C at 800 x g for 20 minutes. After the centrifugation, mononuclear cells in the intermediate fraction were collected and washed three times with PBS, followed by being suspended in DMEM medium supplemented with 10% FCS, which mononuclear cells were used as effector cells.

(11) Separation of Human Complement-containing Serum

Venous blood from a healthy individual was collected in the absence of an anticoagulant and transferred to a 15 ml tube. The blood was incubated in an incubator at 37°C for 60 minutes and then left to stand at room temperature for 60 minutes, followed by centrifugation at 20°C, at 2500 rpm for 15 minutes after peeling off the clot from the wall of the tube. After the centrifugation, the supernatant serum was recovered and used as a complement-containing serum. The serum heated at 56°C for 30 minutes to inactivate the complement was used as a control.

(12) MTT Assay

To the cells cultured in each well of a 96-well plate, TetraColor ONE (SEIKAGAKU CORPORATION) was added in accordance with the protocol described in the attached instructions, and reaction was allowed to occur under 5% $CO_2$ in an incubator for 3 to 4 hours. After the reaction, the 96-well plate was set in a microplate reader as it was and absorbance at 490 nm (control wavelength: 655 nm) was measured.

(13) Complement-dependent Cytotoxicity Activity

HEK293 cells and HEK293(hdlk) cells were peeled off from the plate by trypsinization and the cells were suspended to a population density of $1 \times 10^5$ cells/ml in DMEM medium supplemented with 10% FCS, which were used as target cells. The cells were inoculated in a gelatin-coated 96-well flat-bottomed plate to a density of $1 \times 10^4$ cells/well, and cultured in the presence of anti-human Dlk antibody 4C4 or 31C4, and rat IgG (0.2, 1.0 and 5.0 $\mu$g/ml), respectively, for 30 minutes.
Then the human serum used as a complement was added to an amount of 25% of the culture medium, and the cells were cultured for 72 hours. After the culturing, absorbance was measured by the MTT assay. The absorbance indicating the number of living cells under CDC activity was calculated by subtracting the mean value of the live cells in the well to which the complement-containing serum was added to the culture medium. Statistical significance test was carried out by the Student's $t$ test.

Huh-7EGFP cells and Huh-7(hdlk) cells were peeled off from the plate by trypsinization and the cells were suspended to a population density of $2 \times 10^5$ cells/ml in DMEM medium supplemented with 10% FCS, which were used as target cells. The cells were inoculated in a 96-well flat-bottomed plate to a density of $1 \times 10^4$ cells/well, and cultured in the presence of anti-human Dlk antibody 4C4 or 31 C4, and rat IgG (0.3, 1, 3, 5 and 10 $\mu$g/ml), respectively, for 30 minutes. Then the human serum used as a complement was added to an amount of 25% of the culture medium, and the cells were cultured for 72 hours. After the culturing, absorbance was measured by the MTT assay. The absorbance indicating the number of living cells under CDC activity was calculated by subtracting the mean value of the live cells in the well in which the complement-containing serum was added to the culture medium. Statistical significance test was carried out by the Student's $t$ test.

(14) Antibody-dependent Cytotoxicity Activity

HEK293 cells and HEK293(hdlk) cells were peeled off from the plate by trypsinization and the cells were suspended to a population density of 2 x $10^5$ cells/ml in DMEM medium supplemented with 10% FCS, which were used as target cells. The cells were inoculated in a gelatin-coated 96-well flat-bottomed plate in an amount of 1 x $10^4$ cells/well, and cultured in the presence of anti-human Dlk antibody 1C1, 4C4 or 31C4, and rat IgG (5 $\mu$g/ml), for 30 minutes. The effector cells were added to the target cells at an effector:target ratio of 20:1, 10:1 and 5:1, respectively, and the cells were cultured under 5% $CO_2$ in an incubator for 72 hours. After the culturing, absorbance was measured by the MTT assay. The absorbance indicating the number of living cells under ADCC activity was calculated by subtracting the mean value of the live cells in the well in which the culture medium alone was added as a control. Significant test was carried out by the Student's *t* test.

(15) Establishment of Cell Line Huh-7 Expressing Human Dlk Derived from Human Liver Cancer

The expression vector (pcDNA-hdlk-Flag) described in "1. Materials and Methods, (1) Isolation of Full Length Human dlk cDNA and Construction of Expression Vector" in Example 1, in which the full length cDNA of human Dlk was inserted, was introduced into cells of the cell line Huh-7 derived from human liver cancer (obtained from Laboratory of Cell Growth and Differentiation, Institute of Molecular and Cellular Biosciences, The University of Tokyo), and after selection with G418 (geneticin, GIBCO BRL), two types of cell lines Huh-7(hDlk) (clones PC14 and PC16) which stably express human Dlk were established. As a control, a cell line Huh-7 EGFP which stably expresses EGFP was established by introducing an expression vector (PEGFP) in which the full length cDNA of EGFP was incorporated, into Huh-7 cells and by selection with G418.

(16) Separation of Complement-containing Serum

Venous blood was collected from a male Std:Wister/ST rat of 8-week age in the absence of an anticoagulant, and complement-containing serum was separated by the method described in "1. Materials and Methods, (11) Separation of Human Complement-containing Serum" in Example 2.

(17) Study of Tumorigenicity-enhancing Activity of Human Dlk Gene

The cells of the two types of cell line Huh-7(hDlk) (clones PC14 and PC16) stably expressing human Dlk, respectively, and control cells (cell line Huh-7 EGFP stably expressing EGFP) were subcutaneously transplanted to nude mice of 6-week age (Balb/c; nu/nu, female, JAPAN SLC), in an amount of 3 x $10^6$ cells/100 $\mu$L (PBS:EHS-gel = 1:1), respectively. To investigate the influence by human Dlk gene on the tumorigenicity, control cells were subcutaneously transplanted to one of the left and right side regions in the back of the same individual of nude mouse, and the cells of clone PC14 or PC16 were subcutaneously transplanted to the other region. For 3 weeks from the transplantation, tumor formation of the respective transplanted liver cancer cells was observed. The volume of a tumor was measured in accordance with a conventional method using a caliper and calculated according to the equation:

$$\text{Tumor Volume (mm}^3) = \pi/6 * \text{longer diameter} * (\text{shorter diameter})^3$$

2. Results

The results of "(1) Expression of Human Dlk in Human Normal Liver", "(2) Anti-Dlk Monoclonal Antibody", "(3) Expression of Human Dlk in Cell Line Derived from Human Liver Cancer", and "(4) Expression of Human Dlk in Human Liver Cancer Tissue" were as described in Example 1.

(5) FACS Analysis of 293(hdlk) Cells Using Anti-human Dlk Monoclonal Antibody (Confirmation of Expression Amount of Dlk)

Using the prepared anti-human monoclonal antibody (clone 4C4), FACS analysis was performed on the HEK293 cells and HEK293(hdlk) cells. It was confirmed that Dlk was not expressed on HEK293 cells at all, but was strongly expressed on HEK293(hdlk) cells (Fig. 7).

(6) CDC Activity Using Anti-Human Dlk Monoclonal Antibodies

The fact that Dlk is expressed on human cancer cell lines and cancer tissues suggest the possibility that Dlk may be used as a tumor marker and an anti-human Dlk monoclonal antibody may be used as a therapeutic antibody targeting cancer cells expressing Dlk. Thus, first, cytotoxicity by the antibody and complement, that is, CDC activity was measured (Fig. 8, Tables 3.1 and 3.2). HEK293 cells or HEK293(hdlk) cells, as target cells, were inoculated in a 96-well plate, and the anti-human Dlk antibody (clone 4C4 or 31 C4 was added to a level of 5 μg/ml) and the complement-containing serum were added, followed by culturing the cells. Three days after the beginning of the culturing, injury of the target cells were assayed by the MTT assay. As for the injury of the HEK293(hdlk) cells, the absorbance was decreased and 70 to 90 % decrease in the number of live cells were observed in the system where the anti-Dlk antibody was added to a level of 5 μg/ml, when compared with the system where no antibody was added or the control IgG antibody was added. In cases where the culturing was performed in the medium supplemented with the inactivated complement-containing serum, the absorbance of the system to which the anti-human Dlk antibody (clone 31 C4) was added to a level of 5 μg/ml was the same as the system to which no antibody was added or the control antibody was added, and the number of live cells was about the same (Fig. 8A, Table 3.1). Further, no antibodies showed cytotoxicity activity against the HEK293 cells not expressing Dlk.

Observation of HEK293(hdlk) cells under a microscope revealed that with the system in which the control IgG antibody was added to the complement-containing serum, or in which the anti-human D1k antibody (clone 31C4) was added to the inactivated complement-containing serum, the cells formed colonies and grew. In contrast, in the system in which the anti-human Dlk antibody (clone 31 C4) was added to the complement-containing serum, most of the cells were dispersed and seemed to be dead. On the other hand, as for HEK293 cells not expressing Dlk, no injured cells were observed even in the system where the anti-human Dlk antibody and the complement-containing serum were added.

Further, the CDC activity on the HEK293(hdlk) cells when the anti-human Dlk antibody (clone 4C4 or 31 C4) was added to a level of 0.2, 1.0 or 5 μg/ml) was examined (Fig. 8B, Table 3.2). By measuring the CDC activity three days after the beginning of the culturing by MTT assay, it was confirmed that the number of live HEK293(hdlk) cells decreased in a dose-dependent manner of anti-human Dlk antibody, and that the activity of 31 C4 was higher than that of 4C4. These results indicate that the prepared anti-human Dlk antibodies have a CDC activity against the cells expressing Dlk antigen.

Table 3.1

Absorbance ±SE

| Cell Line | Serum | | | | Inactivated Serum | | |
|---|---|---|---|---|---|---|---|
| | None | 4C4 | 31C4 | Rat IgG | None | 31C4 | Rat IgG |
| HEK293 | 1.12±0.06 | 1.13±0.04 | 1.03±0.05 | 1.11±0.11 | 1.24±0.05 | 1.13±0.05 | 1.12±0.05 |
| HEK293 [hdlk] | 0.43±0.02* | 0.12±0.01* | 0.04±0.00* | 0.43±0.01 | 0.89±0.05 | 0.95±0.02 | 0.75±0.03 |

Table 3.2

Absorbance ±SE

| Anti-DIK antibody | Antibody Level (μg/ml) | | | |
|---|---|---|---|---|
| | None | 0.2 | 1.0 | 5.0 |
| 4C4 | 0.43±0.02* | 0.49±0.00 | 0.52±0.04 | 0.12±0.01* |
| 31C4 | 0.43±0.02* | 0.48±0.02 | 0.33±0.00* | 0.04±0.00* |
| Rat IgG | 0.43±0.02 | 0.43±0.01 | 0.42±0.02 | 0.43±0.01 |

(7) ADCC Activities Using Anti-human Dlk Monoclonal Antibodies

Then the ADCC activities of the prepared anti-human Dlk monoclonal antibodies were measured using the HEK293(hdlk) cells expressing human Dlk as target cells, and using mononuclear cells in the peripheral blood from a healthy individual as effector cells.

In a 96-well plate, HEK293 or HEK293(hdlk) cells were cultured together with the anti-human Dlk monoclonal antibody (clone 1C1, 4C4 or 31C4) and human peripheral blood mononuclear cells, and three days later, the injury of the target cells in each well was measured by MTT assay. The effector:target ratio was 20:1, 10:1 or 5:1. When the effector:target ratio was 10:1, the activity on HEK293(hdlk) cells,

where any of the anti-human Dlk antibodies was added, was similar to the cases

where no antibody was added or the control antibody was added, and the activity on HEK293 cells was also similar (Fig.

9, Table 4). In cases where the effector:target ratio was 20:1 or 5:1, the target cells were not killed in the system where the anti-human Dlk antibody was added. The cytotoxicity on HEK293(hdlk) cells by the effector cells through the any of the anti-human Dlk monoclonal antibodies was not observed.

Table 4

| Cell Line | Effector: Target Ratio =10 | | | | |
|---|---|---|---|---|---|
| | None | 1C1 | 4C4 | 31C4 | Rat IgG |
| HEK293 | 0.64±0.02 | 0.78±0.01 | 0.67±0.04 | 0.58±0.02 | 0.76±0.05 |
| HEK293[hdlk] | 0.45±0.01 | 0.60±0.04 | 0.50±0.01 | 0.52±0.01 | 0.62±0.03 |

Cytotoxicity by the antibody and complement, that is, CDC activity was measured using Huh-7EGFP cells and Huh-7(hdlk) cells (clones PC14 and PC16) (Fig. 11, Table 5). The cells were inoculated in a 96-well plate, and the anti-human Dlk antibody (clone 4C4 or 31C4 was added to a level of 5 μg/ml) and the complement-containing serum were added, followed by culturing the cells. Three days after the beginning of the culturing, injury of the target cells were assayed by the MTT assay. As for the injury of the Huh-7(hdlk) cells, the absorbance was decreased and 24 to 94% decrease in the number of live cells were observed in the system where the anti-Dlk antibody (clone 4C4 or 31 C4) was added, when compared with the system where no antibody was added or the control IgG antibody was added. No antibodies showed cytotoxicity activity against the Huh-7 EGFP cells not expressing Dlk (Fig. 11A, Table 5A).

Further, the CDC activities on Huh-7(hdlk) cells where the anti-human Dlk antibody (clone 4C4 or 31C4) was added to a level of 0.3, 1, 3, 5 or 10 μg/ml were examined (Fig. 11B, Table 5B). Measurement of CDC activities by MTT assay at three days after the beginning of the culturing revealed that both of the antibodies 4C4 and 31 C4 killed Huh-7(hdlk) cells dose-dependently.

Table 5

A

| Cell Line | Absorbance ±SE | | |
|---|---|---|---|
| | Rat IgG | 4C4 | 31C4 |
| Huh-7 EGFP | 1.91 ± 0.04 | 1.64 ± 0.03 | 1.80 ± 0.04 |
| Huh-7 PC14 | 1.77 ± 0.14* | 0.67 ± 0.04* | 0.11 ± 0.00* |
| Huh-7 PC16 | 1.57 ± 0.05* | 1.19 ± 0.15 | 0.56 ± 0.08* |

B

| Cell Line | Ani-Dlk antibody | Absorbance ±SE | | | | | |
|---|---|---|---|---|---|---|---|
| | | Antibody Level (μg/ml) | | | | | |
| | | None | 0.3 | 1.0 | 3.0 | 5.0 | 10.0 |
| EGFP | Rat IgG | 1.89 ±0.04 | 1.87 ±0.07 | 1.87 ±0.06 | 1.87 ±0.04 | 1.91 ±0.04 | 1.86 ±0.08 |
| | 4C4 | 1.89 ±0.04 | 1.74 ±0.12 | 1.86 ±0.08 | 1.76 ±0.03 | 1.64 ±0.03 | 1.78 ±0.07 |
| | 31C4 | 1.89 ±0.04 | 1.47 ±0.07 | 1.74 ±0.04 | 1.82 ±0.06 | 1.80 ±0.04 | 1.74 ±0.08 |
| PC14 | Rat IgG | 1.69 ±0.10 | 1.71 ±0.10 | 1.75 ±0.03 | 1.92 ±0.07 | 1.77 ±0.14 | 1.90 ±0.03 |
| | 4C4 | 1.69 ±0.10* | 1.40 ±0.11 | 1.70 ±0.07 | 1.12 ±0.04* | 0.67 ±0.05* | 0.22 ±0.02* |
| | 31C4 | 1.69 ±0.10* | 1.69 ±0.10 | 1.68 ±0.07 | 0.30 ±0.04* | 0.11 ±0.03* | 0.06 ±0.01* |
| PC16 | Rat IgG | 1.70 ±0.03 | 1.75 ±0.10 | 1.62 ±0.06 | 1.70 ±0.09 | 1.57 ±0.05 | 1.62 ±0.05 |
| | 4C4 | 1.70 ±0.03* | 1.65 ±0.06 | 1.83 ±0.08 | 1.57 ±0.02 | 1.19 ±0.15 | 0.93 ±0.03* |

(continued)

B

| | | Absorbance ±SE | | | | | |
| | | Antibody Level (μg/ml) | | | | | |
| Cell Line | Ani-Dlk antibody | None | 0.3 | 1.0 | 3.0 | 5.0 | 10.0 |
| | 31C4 | 1.70 ±0.03* | 1.67 ±0.04 | 1.67 ±0.08 | 1.00 ±0.07* | 0.56 ±0.08* | 0.26 ±0.01* |

The anti-human Dlk monoclonal antibodies showed complement-dependent cytotoxicity against the cells of Huh-7 cell line derived from human liver cancer, by which the cells were killed. Since expression of Dlk is observed in the cancerous parts of human liver cancer cells, the prepared monoclonal antibodies are effective as therapeutic antibodies which kill the liver cancer cells expressing Dlk. As for the anti-human dlk monoclonal antibody clone 1C1, although neither the CDC activity nor ADCC activity has been observed, since it strongly recognizes the Dlk antigen on the cells of the human liver cancer cell lines and on the liver cancer pathologic sections as described in "2. Results (4) Expression of Human Dlk in Human Liver Cancer Tissue", and since either the ADCC activity or CDC activity depends on the constant region (Fc) of the antibody, an anti-dlk monoclonal antibody which exerts anticancer action may be prepared by forming a chimeric antibody or humanized antibody in which at least the constant region is derived from human Fc.

(8) FACS Analysis of Huh-7(hdlk) Cells Using Anti-human Dlk Monoclonal Antibody (Confirmation of Expression Amount of Dlk)

Using the prepared anti-human monoclonal antibody, FACS analysis was performed on the Huh-7EGFP cells and Huh-7(hdlk) cells, by the method described in "1. Materials and Methods, (9) FACS Analysis" in Example 1. It was confirmed that human Dlk was not expressed on Huh-7EGFP cells at all, but was strongly expressed on Huh-7(hdlk) cells (Fig. 10).

(9) Enhancement of Tumorigenicity by Expression of Human Dlk Gene

Although it was proved that Dlk is expressed on human liver cancer cell lines and liver cancer tissues, the function of Dlk in the formation of liver cancerous tumor was not clear. Clone PC14 cells of the Huh-7(hDlk) cell line derived from human liver cancer stably expressing human Dlk were subcutaneously transplanted to nude mice and the tumor formation was compared with the case where the control cells (Huh-7 EGFP) were transplanted. As a result, in all of the 5 individuals which received transplantation, drastic growth of tumor by clone PC 14 cells was observed (Fig. 12A). At 19 days from the transplantation, the volume of the cancer tissue in those to which the control cells were transplanted was $1271 \pm 427.5$ (mm$^3$), while that of the cancer tissue in those to which the clone PC14 cells were transplanted was $4319.4 \pm 378.5$ (mm$^3$). Similar experiments were carried out for PC16 cells, and drastic growth of the tumor was observed again when compared with the control cells (Fig. 12B). These results suggest that Dlk has a function to drastically enhance the tumor formation of the liver cancer, and indicate that Dlk is suitable as a target in the therapy of liver cancer.

Industrial Availability

The method for detecting liver cancer and the diagnostic drug for liver cancer are useful for the diagnosis of liver cancer. The therapeutic drug for cancer is useful for therapies of cancers such as liver cancer.

## Claims

1. Use of an antibody which undergoes antigen-antibody reaction with Dlk expressing on surfaces of liver cancer cells and which exerts anticancer action against said liver cancer cells, for the production of a therapeutic drug for the treatment of liver cancer.

2. The use according to claim 1, wherein said liver cancer cells are hepatocellular carcinoma cells and/or cholangiocellular carcinoma cells.

3. The use according to claim 1 or claim 2, wherein said antibody is a monoclonal antibody.

4. The use according to any one of claims 1 to 3, wherein said cancer cells are human cells, and said antibody is an anti-human Dlk antibody.

5. The use according to any one of claims 1 to 4, wherein said antibody exerts anticancer action in the presence of complement.

## Patentansprüche

1. Verwendung eines Antikörpers, der eine Antigen-Antikörper-Reaktion mit Dlk eingeht, das auf Oberflächen von Leberkrebszellen exprimiert wird, und der eine Antikrebs-Wirkung gegen die Leberkrebszellen ausübt, zur Produktion eines therapeutischen Medikaments zur Behandlung von Leberkrebs.

2. Verwendung nach Anspruch 1, worin die Leberkrebszellen Leberzellkarzinom-Zellen und/oder Cholangiom-Zellen sind.

3. Verwendung nach Anspruch 1 oder Anspruch 2, worin der Antikörper ein monoklonaler Antikörper ist.

4. Verwendung nach einem der Ansprüche 1 bis 3, worin die Krebszellen menschliche Zellen sind und der Antikörper ein Anti-Human-Dlk-Antikörper ist.

5. Verwendung nach einem der Ansprüche 1 bis 4, worin der Antikörper Antikrebs-Wirkung in Gegenwart von Komplement ausübt.

## Revendications

1. Utilisation d'un anticorps qui subit une réaction antigène - anticorps avec Dlk s'exprimant sur les surfaces de cellules du cancer du foie et qui exerce une action anti-cancéreuse contre lesdites cellules de cancer du foie, pour la production d'un médicament thérapeutique pour le traitement du cancer du foie.

2. Utilisation selon la revendication 1, dans laquelle lesdites cellules de cancer du foie sont des cellules de carcinome hépatocellulaire et/ou des cellules de cholangiome.

3. Utilisation selon la revendication 1 ou la revendication 2, dans laquelle ledit anticorps et un anticorps monoclonal.

4. Utilisation selon l'une quelconque des revendications 1 à 3, dans laquelle lesdites cellules de cancer sont des cellules humaines, et ledit anticorps est un anticorps anti-Dlk humaine.

5. Utilisation selon l'une quelconque des revendications 1 à 4, dans laquelle ledit anticorps exerce une action anti-cancéreuse en présence du complément.

Muscle Week 12
Kidney Week 12
Liver Week 12
Lung Week 12
Heart Week 12
Brain Week 12
Fetus Week 12

Probe : hdlk

(B)

▼ human dlk

Week 12
Week 10
Week 9
Week 8
Week 7
Week 6

28s
18s

(A)

Peripheral Leukocytes
Lung
Placenta
Small Intestine
Liver
Kidney
Spleen
Thyroid
Colon
Skeletal Muscle
Heart
Brain

(C)

Fig. 1

Fig. 2

**Fig. 3**

A

B

Fig. 4

EP 1 702 982 B1

**Fig. 5**

HEStaining  Dlk-positive  Dlk-negative

Grade I

Grade II

Grade III

**Fig. 6**

HEK293          HEK293(hdlk)

**Fig. 7**

A

B

Fig. 8

Fig. 9

Fig. 10

EP 1 702 982 B1

Fig. 11

**B**

Tumor Volume (mm³)

4000
3500
3000
2500
2000
1500
1000
500
0

Mouse 1(EGFP)
Mouse 1 (PC16)
Mouse 2 (EGFP)
Mouse2(PC16)
Mouse3(EGFP)
Mouse3(PC16)
Mouse 4 (EGFP)
Mouse 4 (PC16)

6  12  13  14  15  16  19  21

**Days after Transplantation**

**A**

Tumor Volume (mm³)

6000
5000
4000
3000
2000
1000
0

Mouse 1(EGFP)
Mouse 1(PC14)
Mouse 2 (EGFP)
Mouse2(PC14)
Mouse3(EGFP)
Mouse 3(PC14)
Mouse 4 (EGFP)
Mouse 4(PC14)
Mouse 5 (EGFP)
Mouse 5(PC14)

5  7  11  13  15  18  19

**Days after Transplantation**

**Fig. 12**

36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02103033 A
- EP 04819413 A
- JP 2004017499 W
- JP 2003399331 A
- JP 2003401585 A
- JP 2003423237 A

### Non-patent literature cited in the description

- **Laborda, J. et al.** *J. Biol .Chem.,* 1993, vol. 268 (6), 3817-20
- **Smas, C.M. et al.** *Cell,* 1993, vol. 73 (4), 725-34
- **Floridon, C. et al.** *Differentiation,* 2000, vol. 66 (1), 49-59
- **Harken, J.C. et al.** *Tumour Biol.,* 1999, vol. 20 (5), 256-62
- **Jensen, C.H. et al.** *Br. J. Dermatol.,* 1999, vol. 140 (6), 1054-9
- **Ohno, N. et al.** *Stem Cells,* 2001, vol. 19 (1), 71-9
- **Tanimizu, N. et al.** *J. Cell Sci.,* 2003, vol. 116, 1775-86
- **Onishi, M. et al.** *Exp. Hematol.,* 1996, vol. 24, 324-329
- **Sell, S.** *Int. J. Dev. Biol.,* 1993, vol. 37, 189-201
- **Jensen, C.H. et al.** *Eur. J. Biochem.,* 1994, vol. 225, 83-92
- **Kaneta, M. et al.** *J. Immunol.,* 2000, vol. 164, 256-264
- **Okada, S. et al.** *Oncology,* 1993, vol. 50 (1), 22-26
- **Kitajima, T. et al.** *Nat. Biotechnol.,* 1999, vol. 17 (5), 487-490
- **Jensen, C. H. et al.** *Br. J. Dermatol.,* 1999, vol. 140 (6), 1054-1059
- **Russell, W. C. et al.** *J. Gen. Virol.,* 1977, vol. 36, 59-72
- **Kipps, T. J. et al.** *J. Exp. Med.,* 1985, vol. 161, 1-17
- **Pear, W.S. et al.** *Proc. Natl. Acad. Sci. USA,* 1993, vol. 90, 8392-8396
- **Yanai,N. et al.** *Exp. Cell Res.,* 1991, vol. 197, 50-56
- **Larsen, J.B. et al.** *Lancet.,* 1996, vol. 347, 191
- **Jensen, C.H. et al.** *Hum. Reprod.,* 1993, vol. 8, 635-641